# EUROPEAN PATENT SPECIFICATION

(11) **EP 1 791 961 B1**
(45) Date of publication and mention of the grant of the patent: **08.06.2011**
(21) Application number: 05774845.1
(22) Date of filing: 12.08.2005
(51) Int. Cl.: C12N 15/70, C12P 21/00, C07K 19/00

(54) **PROTEIN PRODUCTION METHOD UTILIZING YEBF**
PROTEINHERSTELLUNGSVERFAHREN UNTER NUTZUNG VON YEBF
PROCEDE DE PRODUCTION D'UNE PROTEINE UTILISANT LA PROTEINE YEBF

(30) Priority: 18.08.2004 US 522125 P
(43) Date of publication of application: 06.06.2007
(73) Proprietor: The Governors of the University of Alberta, Edmonton, AB T6G 2E1 (CA)
(72) Inventor: WEINER, Joel, Edmonton, Alberta T6J 2C2 (CA); ZHANG, Guijin, Edmonton, Alberta T6R 3B4 (CA)
(74) Representative: Ouzman, Beverley Nicola Claire
(86) International application number: PCT/CA2005/001249
(87) International publication number: WO 2006/017929

(56) References cited:
- WO-A-00/66756
- US-B1- 6 733 997
- DYK VAN T K ET AL: "A genomic approach to gene fusion technology" PROCEEDINGS OF THE NATIONAL ACADEMY OF SCIENCES OF USA, NATIONAL ACADEMY OF SCIENCE, WASHINGTON, DC.; US, vol. 98, no. 5, 27 February 2001 (2001-02-27), pages 2555-2560, XP002285714 ISSN: 0027-8424
- BATISSON I ET AL: "Full capacity of recombinant Escherichia coli heat-stable enterotoxin fusion proteins for extracellular secretion, antigenicity, disulfide bond formation, and activity" INFECTION AND IMMUNITY, AMERICAN SOCIETY FOR MICROBIOLOGY. WASHINGTON, vol. 68, no. 7, 1 July 2000 (2000-07-01), pages 4064-4074, XP002171524 ISSN: 0019-9567
- POQUET I. ET AL.: 'Stable periplasmic secretion intermediate in the general secretory pathway of Escherichia coli.' THE EMBO J. vol. 12, no. 1, January 1993, pages 271 - 278, XP008113934
- BRAUN P. ET AL.: 'Proteome-scale purification of human proteins from bacteria' PROC NATL ACAD SCI USA vol. 99, no. 5, 05 March 2002, pages 2654 - 2659, XP008113935
- SHOKRI A. ET AL.: 'Cell and process design for targeting of recombinant protein into the culture medium of Escherichia coli.' APPL MICROBIOL BIOTECHNOL vol. 60, 2003, pages 654 - 664, XP009078758
- BROKX SJ ET AL.: 'Genome-wide analysis of lipoprotein expression in Escherichia coli MG1655' J. BACTERIOL vol. 86, no. 10, May 2004, pages 3254 - 3258, XP008113936

## Description

This application claims priority to US provisional application no. 60/522,125.

### FIELD

The present invention relates to the field of secretion of proteins by host cells, into a growth medium.

### BACKGROUND

Protein secretion from bacteria plays an important role in many aspects of the bacterial life cycle, including the formation of pili and flagella, the secretion of extracellular enzymes to digest polymers for nutritional purposes and the secretion of toxins to kill host cells in infections of humans, animals and plants. It is generally accepted that non-pathogenic laboratory strains of *E. coli,* particularly K12 strains, do not naturally secrete proteins into the extracellular medium under routine growth conditions^{6,8}, although gene sequence analysis implies the possible existence of a protein secretion pathway⁶. Pugsley has postulated that this system has been lost as a result of prolonged laboratory passage and storage over many decades⁶.

*Bacillus subtilis* has a well characterized protein excretion system for extracellular enzymes such as α-amylase and subtilin and this organism has been used as an alternative for the extracellular production of recombinant proteins⁷. Compared to *E. coli,* however, *B. subtilis* has several disadvantages including plasmid instability, lack of suitable expression control systems and degradation of the recombinant proteins in the culture medium by the high level of proteolytic enzymes that are also secreted from these cells⁷. In addition, compared with *E. coli,* a relatively long culture period is required for *B. subtilis.*

Other investigators have examined the secretion of proteins in *E*. *coli.* He *et al.*¹⁰ introduced a cluster of *out* genes (~12kb) carried on a cosmid to *E. coli* cells whose chromosome had *Erwinia* pectate lyase gene integrated. The *out* genes encode the secretion apparatus of *Erwinia chrysnthemi,* an enterobacterial plant pathogen. With the help of *out* gene expression, *E. coli* could secrete the enzyme to the medium. Pugsley's group⁸ has shown that *E. coli* cells can secrete *Klebsiella oxytoca* pullulanase into the medium by co-expressing 14 *Klebsiella* secretion machinery genes cloned on a second plasmid. Pugsley's group¹¹ has also shown that the secretion of an endogenous *E. coli* chitinase into the medium can occur by co-expressing a cluster of *E*. *coli gsp* genes carried on a second plasmid in PAP5066, an *hns*-inactivated *E. coli* K12 strain. The *gsp* genes encode the putative type II secretion machinery proteins of *E. coli* and are silenced by H-NS protein, a global regulator and nucleoid-structuring protein¹¹. Overall, these excretion studies required the artificial induction of secretion machinery proteins.

In *E. coli* K12, there are 2 well-identified protein transport pathways across the cytoplasmic membrane- the sec-dependent pathway or the general secretion pathway (GSP) and the sec-independent Mtt/Tat pathway¹². The GSP of gram-negative bacteria, has at least six different terminal branches depending on the secretion pathway in the outer membrane¹³. Genomic analysis of *E. coli* has identified genes that are homologous to genes encoding secretion proteins in other bacteria. These include *gsp* and *yacC* (*gspS*) homologous to *Klebsiella pulS*¹¹*,* found at 74.5 and 2.95 minutes on the *E. coli* chromosome, respectively. Although these genes appear to encode functional proteins¹⁴ their transcription is turned off under standard growth condition⁶. g*sp* genes, for example, are silenced by H-NS as mentioned above¹¹.

*E. coli* is the most widely used bacterium for protein expression both in research and in industry. As it is desirable to have a secretion system in common laboratory strains, investigators have explored different ways of producing recombinant proteins in the medium^{5, 37, 38}. The accumulation of recombinant proteins in the culture medium has the potential to offer a number of benefits including, increased protein production and purity, reduction in cellular toxicity of over-expressed proteins, avoidance of the formation of inclusion bodies and protein degradation by cytoplasmic proteolytic enzymes. It has also been found that proper recombinant protein folding is enhanced by the more favorable redox potential in the medium⁵.

Based on sequence motif algorithms, the *E*. *coli yebF* gene is predicted to encode a small lipoprotein (Blattner B 1847 or Swiss-Prot P33219) of unknown function attached to the membrane. The *yebF* gene is part of the *yebGFE* operon that is negatively regulated by LexA^{1,9}. *yebF* gene expression has been observed under several stress conditions including UV irradiation² and DNA damage induced by mitomycin C^{3,9}. However, little else has been reported.

### SUMMARY

YebF is a small (10.8 kD) soluble endogenous protein that is naturally secreted into the culture medium by *E. coli* cells. Commonly used laboratory strains HB101 (a hybrid of *E. coli* K12 and B strains), BL21(DE3) (a B strain) and MG1655 (a K12 strain) secrete YebF into the medium under standard laboratory conditions.

YebF can be used to transport proteins into the growth medium. Fusion proteins comprising YebF and human interleukin-2 (hIL2), the short form of *B. subtilis* X23 α-amylase gene⁸ lacking the signal sequence and leaderless *E. coli* alkaline phosphatase, have been expressed and secreted in *E. coli.* Therefore, YebF can be used to direct the secretion of proteins, polypeptides and peptides into the medium. Further, the secreted fusion proteins are active.

hIL2 is a 15kD hydrophobic protein¹⁵, the short form of α-amylase is a 48 kD hydrophilic protein, and leaderless *E. coli* alkaline phosphatase is a hydrophilic protein. Therefore, YebF can direct the secretion of both hydrophobic and hydrophilic proteins to the medium. Further, YebF can carry proteins of varying size to the medium. However, it was noted that the secreted YebF-hIL2 fusion was sensitive to proteolytic degradation in the medium.

When the fusion protein is further engineered to comprise a peptide tag, such as a His6 tag, the secreted fusion protein can be readily purified from the medium, for example by affinity chromatography, or it can be readily identified, for example with a fluorescent antibody. Expression of a fusion protein into the culture medium provides a starting material for purification wherein the secreted fusion protein is relatively pure, as compared to proteins expressed in the cytoplasm. Therefore subsequent purification steps may be simpler and less expensive. Further, production of secreted fusion proteins in the medium could reduce the contamination of these proteins with lipopolysaccharides in the purified product¹⁶.

Therefore, in one aspect, this invention is a method of producing a protein, polypeptide or peptide of interest in a suitable bacterial cell that comprises:
(a) providing an expression vector that encodes a fusion protein comprising YebF including the leader sequence to direct the protein, polypeptide or peptide of interest to the periplasm, fused amino-terminally to the protein, polypeptide or peptide of interest, and
(b) expressing the fusion protein in the bacterial cell to thereby generate a secreted fusion protein.

The bacterial cell can be *Escherichia coli.* The method may further comprise the step of purifying a secreted fusion protein from the medium in which the bacterial cell is growing. The fusion protein may further comprise at least one tag or at least one protein cleavage site.

In another aspect, the invention is the use of YebF to direct the secretion of a protein, polypeptide or peptide of interest into bacterial growth medium, wherein the YebF is fused amino-terminally to the protein, polypeptide or peptide of interest.

In another aspect, the invention is the use of the leader sequence of YebF to direct transport of a protein, peptide or polypeptide of interest to the periplasm, wherein the leader sequence of YebF is fused amino-terminally to the protein, polypeptide or peptide of interest.

In yet another aspect, the invention is a method for expressing a protein, polypeptide or peptide of interest in the periplasm comprising:
a) providing an expression vector that encodes a fusion protein comprising the protein, polypeptide or peptide of interest located carboxy-terminally to the leader sequence of YebF, and
b) expressing the protein in a suitable bacterial cell.

The nucleotide sequence that encodes the protein, polypeptide or peptide of interest may be located downstream of the nucleotide sequence that encodes the leader sequence of YebF. The bacterial cell may be *Escherichia coli.* The fusion protein may further comprise at least one tag.

### BRIEF DESCRIPTION OF THE DRAWINGS

**Figure 1****.** Alignment of the leader sequences of *E. coli* YebF protein and homologues from other bacteria. *E. coli* (SEQ ID NO: 14); *S. flexneri* (SEQ ID NO: 15); *S. typhi* (SEQ ID NO: 16); *S. typhimurium, P. luminescens* (SEQ ID NO: 18).
**Figure 2****.** Western blot demonstrating the subcellular localization of *E. coli* YebF and mature YebF. Symbols: S, spheroplasts; P, periplasm; H, hypertonic solution; M, medium (Panel A). Western blot demonstrating secretion of mature YebF protein from *E. coli* cells over time. Lanes 1-7 show the cells (0 - 180 min) and lanes 8-14 show the medium (0 - 180 min) at 30 min time intervals. Lane F is purified YebF-His₆ (Panel B)
**Figure 3****.** Protein profiles in the medium from HB101 cells harbouring pMS119EH (Ctr lanes) or pYebFH₆/MS (YebF lanes). Samples were collected at the times indicated after induction with 0.1 mM IPTG. Proteins were separated on a 10% SDS-Tricine gel.
**Figure 4****.** Maldi-TOF linear mode mass spectrometry of purified *E. coli* YebF protein with a 6-histidine tag.
**Figure 5****.** Western blot demonstrating localization of YebF and mature YebF and CRP in cells (C) or medium (M), over time. YebF and mature YebF were detected with anti-His antibody (Panel A), CRP was detected with anti-CRP antibody (Panel B). Lane F in panel A is the purified YebF-His₆.
**Figure 6****.** Panel A: α-amylase activity of the various fluids and subcellular fractions of HB101 cells harboring: (a) a short form α-amylase gene fused to *yebF* gene (pYebF-AmyH₆), (b) a short form α-amylase gene fused to the sec-leader sequence of *yebF* (pLS-AmyH₆), and (c) a short form α-amylase gene (pAmyH₆). Data is the average of three replicates with the standard deviation shown.
   Panel B: Protein profiles in the medium. HB101 cells harbouring pAmyH₆ (A lanes), pLS-AmyH₆ (B lanes) and pYebF-AmyH₆ (C lanes) were grown in TB. Protein samples from the medium harvested at the indicated time after induction were prepared as in Methods and Materials. Proteins were separated on 10% SDS-PAGE.

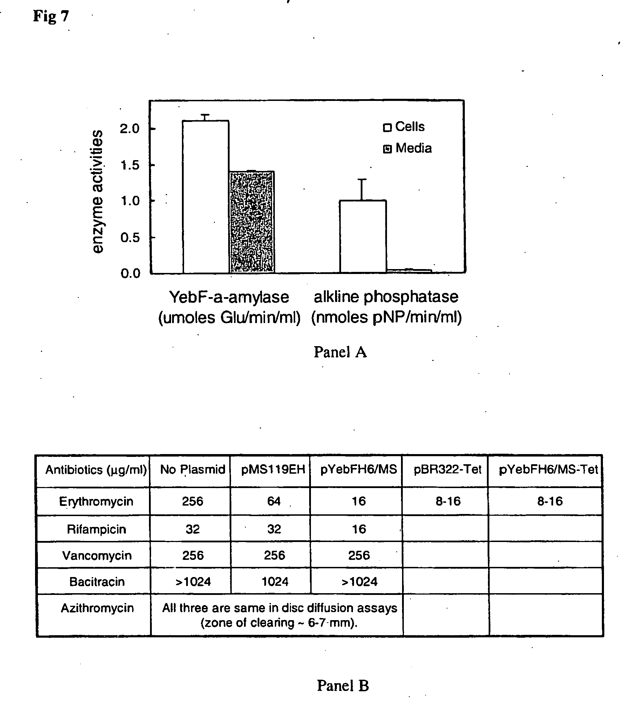

**Figure 7****.** Distribution of YebF-α-amylase and alkaline phosphatase, a periplasmic enzyme. Activities shown are the average of two determinations (Panel A). MIC (Minimum Inhibitory Concentrations) test of HB101 cells harboring the plasmids for the antibiotics indicated in the Table. (Panel B) pBR322-tet and pYebFH₆/MS-tet are tetracycline resistant. The remainder are amp-resistant.

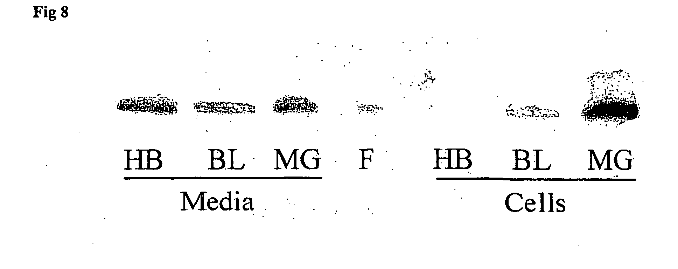

**Figure 8****.** Western blotting demonstrating secretion of mature YebF from *E. coli* HB101/pYebFH₆/MS (HB), BL21(DE3)/pYebFH₆/T7 (BL) and MG1655/pYebFH₆/MS (MG) cells. Lane F, purified YebF-His₆.

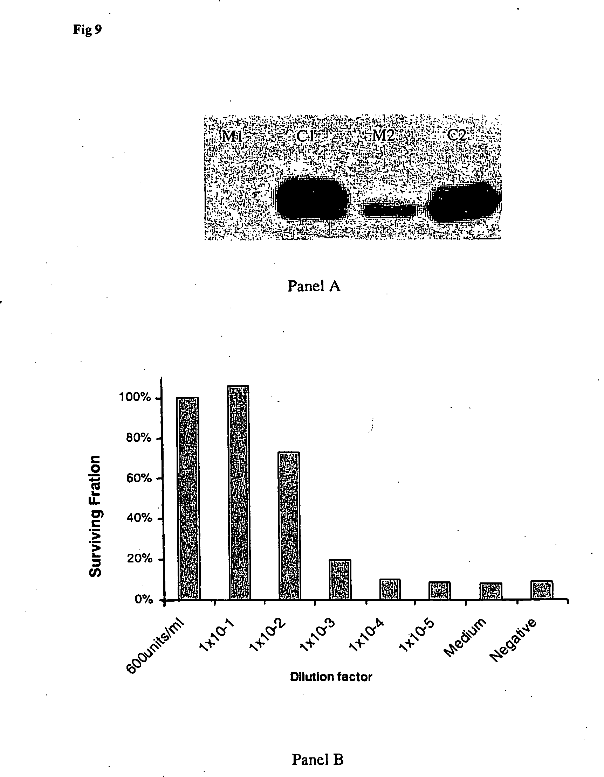

**Figure 9****.** Western blotting demonstrating the secretion of mature YebF-hIL2 fusion protein from HB101 harboring pYebF-hIL2H₆/MS. Panel A: shows the localization of YebF-hIL2 by immunoblotting using an anti-6xhistidine tag antibody. Lane M1, medium + 10mM DTT; C1, cells + 10mM DTT; M2, medium - DTT; C2, cells - DTT.
   Panel B shows the activities of the secreted mature YebF-hIL2 fusion protein in the diluted medium. The bar labeled "600 units/ml" corresponds to standard hIL-2 with the well containing 50 µl of 600 units of hIL-2/ml in RHFM medium (see Materials and Methods) and 50 µl of CTLL-2 cells. The bar labeled "negative" is a negative control in which RHFM without hIL-2 was used. The bar labeled "medium" corresponds to ten times diluted medium from HB101/pMS119EH. The *E. coli* culture medium harvested 3 hours after induction was filtered through a 0.22 µm Millipore filter and then diluted with RHFM at the ratios indicated. Each bar was averaged from triplicate wells.

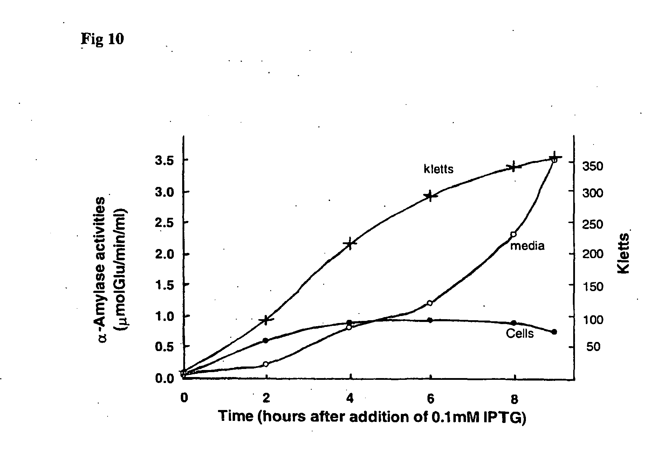

**Figure 10****.** Secretion of mature YebF-α-amylase fusion protein over time from HB101 harboring pYebF-AmyH₆. Samples were taken at 2 hour intervals following 0.1 mM IPTG induction and assayed for α-amylase activity. Growth was monitored with a Klett spectrophotometer equipped with a red filter. α-amylase activity in the cells and the medium was expressed in units per ml of the original culture.

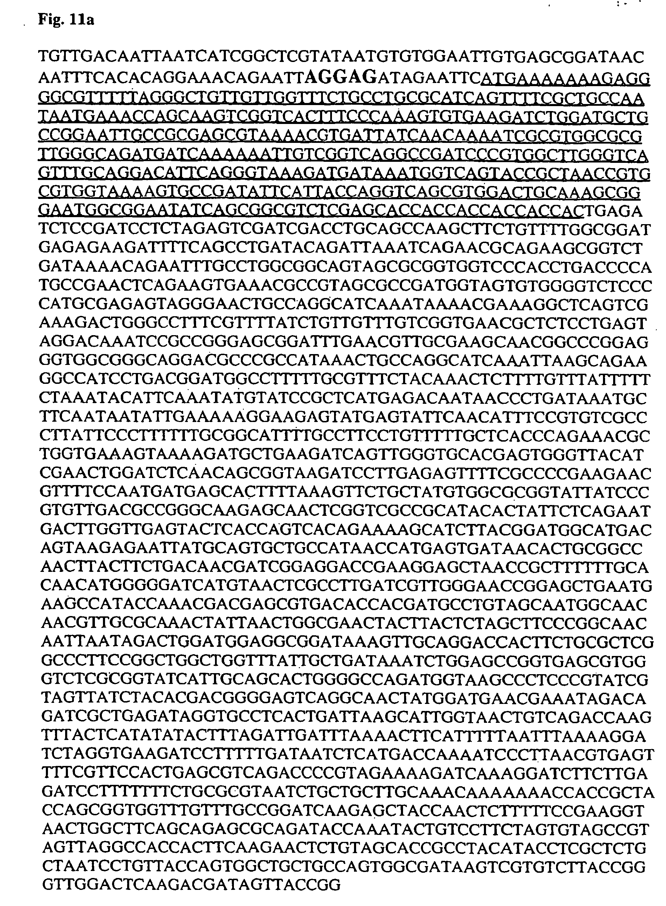

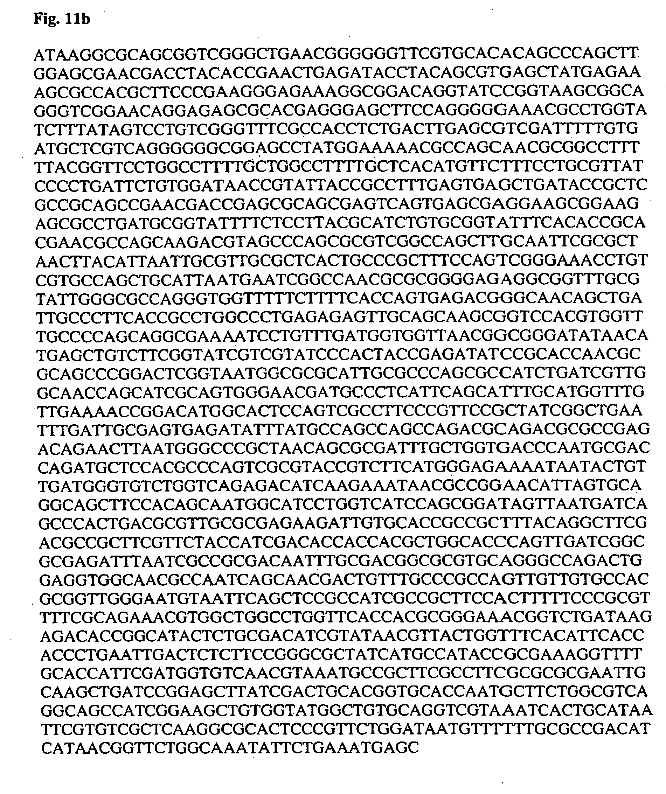

**Figure 11****.** The sequence of pYebFH6/MS (SEQ ID NO: 19). The ribosome binding site is in bold, and the YebF coding region with a 6-histidine tag is underlined.

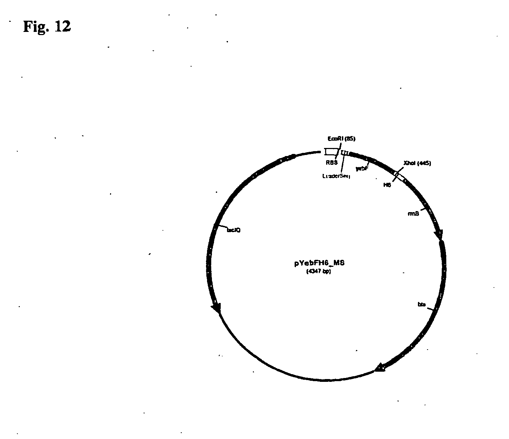

**Figure 12****.** The physical map of pYebFH6/MS.

### DETAILED DESCRIPTION

In accordance with the aspects of the present invention described above, there is provided a method for secreting a protein, polypeptide or peptide of interest into bacterial growth medium. Also disclosed herein is a method of producing a protein, polypeptide or peptide of interest. The method utilizes YebF to direct or cause the protein, polypeptide or peptide of interest, to be secreted as a fusion protein into the bacterial growth medium. The fusion protein comprises YebF fused to a protein, polypeptide or peptide of interest. The fusion protein may optionally be tagged and may optionally comprise an amino acid sequence that permits cleavage of the YebF encoded portion and/or the tag, from the protein, polypeptide or peptide of interest.

To facilitate understanding of the invention, a number of terms are defined below.

The term "nucleotide" refers to a ribonucleotide or a deoxyribonucleotide. "Nucleic acid" refers to a polymer of nucleotides and may be single- or double-stranded. "Polynucleotide" refers to a nucleic acid that is twelve (12) or more nucleotides in length.

The term "nucleotide sequence of interest" refers to any nucleotide sequence that encodes a "protein, polypeptide or peptide sequence of interest", the production of which may be deemed desirable for any reason, by one of ordinary skill in the art. Such nucleotide sequences include, but are not limited to, coding sequences of structural genes (*e.g*., reporter genes, selection marker genes, oncogenes, drug resistance genes, growth factor genes, etc.), regulatory genes (*e.g*., genes encoding activator protein 1 (AP1), activator protein 2 (AP2), Sp1, etc.), antibody genes, enzyme genes, etc., or portions thereof. The nucleotide sequence of interest may comprise the coding sequence of a gene from one of many different organisms (*e.g*., mammalian, insect, bacterial, and viral genes).

A nucleotide sequence "encodes" or "codes for" a protein if the nucleotide sequence can be translated to the amino acid sequence of the protein. The nucleotide sequence does not have to contain an actual translation start codon or termination codon.

A "protein, polypeptide or peptide sequence of interest" is encoded by the "nucleotide sequence of interest". The protein, polypeptide or peptide may be a protein from any organism, including but not limited to, mammals, insects, micro-organisms such as bacteria and viruses. It may be any type of protein, including but not limited to, a structural protein, a regulatory protein, an antibody, an enzyme, an inhibitor, a transporter, a hormone, a hydrophilic or hydrophobic protein, a monomer or dimer, a therapeutically-relevant protein, an industrially-relevant protein, or portions thereof.

"Expression" as used herein refers to transcription or translation, or both, as context requires.

"Secretion" as used herein refers to the excretion of the fusion protein that is expressed in a bacterium, into the bacterial growth medium.

"YebF" is a reference to the protein having the following amino acid sequence (SEQ ID NO: 1).

"Mature YebF" is a reference to the protein having the following amino acid sequence (SEQ ID NO: 2)

*yebF* is a reference to a nucleic acid or a nucleotide sequence having the following sequence (SEQ ID NO: 3):

The terms "modified", "mutant" or "variant" are used interchangeably herein, and refer to: (a) a nucleotide sequence in which one or more nucleotides have been added or deleted, or substituted with different nucleotides or modified bases (*e.g*., inosine, methylcytosine) or to (b) a protein, peptide or polypeptide in which one or more amino acids have been added or deleted, or substituted with a different amino acid. A variant may be naturally occurring, or may be created experimentally by one of skill in the art. A variant of YebF or *yebF* may be a protein, peptide, polypeptide or polynucleotide that differs (*i.e*., an addition, deletion or substitution) in one or more amino acids or nucleotides from the sequence of YebF or *yebF,* respectively.

In this regard, it is well understood in the art that certain alterations inclusive of mutations, additions, deletions and substitutions can be made to a reference nucleic acid or protein, whereby the altered nucleic acid or protein retains a particular biological function or activity, or perhaps displays an altered but nevertheless useful activity. Some deletions, insertions and substitutions will not produce radical changes in the characteristics in the YebF protein or in the *YebF* nucleic acid. However, while it may be difficult to predict the exact effect of the substitution, deletion or insertion in advance of doing so, one skilled in the art will appreciate that the effect can be evaluated by routine screening assays. For example whether a variant of YebF has a secretory function can be determined by assaying for whether the YebF variant, or a fusion protein comprising the YebF variant, is secreted into the medium, by the methods disclosed in the Materials and Methods, and Examples disclosed herein. Modifications of protein properties such as redox or thermal stability, hydrophobicity, susceptibility to proteolytic degradation, or the tendency to aggregate with carriers or into multimers may be assayed by methods well known to one of skill in the art.

Variants may be created experimentally using random mutagenesis, oligonucleotide-mediated (or site-directed) mutagenesis, PCR mutagenesis and cassette mutagenesis. Oligonucleotide-mediated mutagenesis is well known in the art as, for example, described by Adelman³¹ using vectors that are either derived from bacteriophage M13, or that contain a single-stranded phage origin of replication as described by Viera et al.³² Production of single-stranded template is described, for example, in Sambrook.¹⁷ Alternatively, the single-stranded template may be generated by denaturing double-stranded plasmid (or other DNA) using standard techniques.

Alternatively, linker-scanning mutagenesis of DNA may be used to introduce clusters of point mutations throughout a sequence of interest that has been cloned into a plasmid vector. For example, reference may be made to Ausubel.³³ Region-specific mutagenesis and directed mutagenesis using PCR may also be employed to construct variants according to the invention. In this regard, reference may be made, for example, to Ausubel.³³ With regard to random mutagenesis, methods include incorporation of dNTP analogs³⁴ and PCR-based random mutagenesis such as described in Stemmer and Shafikhani.^{35,36}

The term "biologically active" when made in reference to a variant or portion of YebF, refers to a protein, polypeptide or peptide possessing at least the secretory function of YebF. A biologically active variant or portion of YebF has at least about 5%, preferably at least about 25%, more preferably at least about 50% and most preferably at least about 75% of the secretory activity of YebF. Whether the variant or portion of the YebF protein has secretory activity may be determined, for example, by the methods disclosed in the Materials and Methods, and Examples disclosed herein. A biologically active variant or portion of YebF may either be secreted into the growth medium, or may effect the secretion a fusion protein (of which it comprises part) into the growth medium, and both of these activities are considered to be a secretory activity.

The term "portion" when used in reference to a protein refers to fragments of that protein. The fragments may range in size from four amino acid residues to the entire amino acid sequence of the protein, minus one amino acid.

A "peptide" is polymer of four to 20 amino acids, a "polypeptide" is a polymer of 21 to 50 amino acids and a "protein" is a polymer of more than 50 amino acids.

A fusion protein is a recombinant protein comprising regions derived from at least two different proteins. The term "fusion protein" as used herein refers to a protein molecule in which a protein, polypeptide or peptide of interest is fused to YebF. "Fused", in one context means that nucleic acid encoding YebF is joined in frame to the nucleic acid encoding the protein, polypeptide or peptide of interest, to provide for a single amino acid chain when transcription and translation occur. In another context, "fused" may also be a reference to the joining of a protein, polypeptide or peptide of interest to YebF.

A "secreted fusion protein" is the part of the fusion protein that is secreted into the bacterial growth medium. As is apparent, a secreted fusion protein will likely lack the amino acids that comprise the leader sequence of YebF, specifically MKKRGA FLGLLLVSAC ASVF (SEQ ID NO: 20).

An "expression vector" refers to a recombinant DNA molecule containing the appropriate control nucleotide sequences (*e.g*., promoters, enhancers, repressors, operator sequences and ribosome binding sites) necessary for the expression of an operably linked nucleotide sequence in a particular host cell. By "operably linked/linking" or "in operable combination" is meant that the nucleotide sequence is positioned relative to the control nucleotide sequences to initiate, regulate or otherwise direct transcription and/or the synthesis of the desired protein molecule.

The expression vector may be self-replicating, such as a plasmid, and may therefore carry a replication site, or it may be a vector that integrates into a host chromosome either randomly or at a targeted site. The expression vector may contain a selection gene as a selectable marker for providing phenotypic selection in transformed cells. The expression vector may also contain sequences that are useful for the control of translation.

"Purified" or "to purify" refers to the removal of undesired components from a sample. For example, to purify the secreted fusion protein from the bacterial growth medium, may mean to remove other components of the medium (*i.e*., proteins and other organic molecules) thereby increasing the percentage of the secreted fusion protein.

The present invention utilizes YebF to direct the secretion of a protein, polypeptide or peptide of interest, into the bacterial growth medium. This may be accomplished by generating a fusion protein, which comprises YebF and the protein, polypeptide or peptide of interest.

A recombinant DNA molecule that encodes the fusion protein can be made, for example, by ligating a nucleic acid that encodes the protein, polypeptide or peptide of interest (a nucleotide sequence of interest), in frame, to a nucleic acid that encodes the YebF protein, or a biologically active variant or portion thereof. In one embodiment, the nucleic acid that encodes the protein, polypeptide or peptide of interest may be ligated to the 3'-end (*i.e*., downstream) of the nucleic acid that encodes the YebF protein. In another embodiment, the nucleic acid that encodes the protein, polypeptide or peptide of interest may not be ligated directly to the 3'-end of the YebF protein. Rather, a spacer nucleotide sequence may separate the 3'-end of the nucleotide sequence encoding the YebF protein, and the 5'-end of the nucleotide sequence that encodes the protein, polypeptide or peptide of interest. The spacer nucleotide sequence encodes one or more amino acids that may or may not be functional (*i.e*., a tag, or a cleavage site, or no function at all but to separate the two parts). Therefore, in the fusion protein produced by these embodiments, the protein, polypeptide or peptide of interest is located carboxy-terminally to the YebF protein. Methods of generating this recombinant DNA molecule are known to those of skill in the art, examples of which are provided by the methods disclosed in the Materials and Methods, and Examples disclosed herein, or the references cited herein.

The nucleotide sequence that encodes the YebF protein, or the nucleotide sequence that encodes the protein, polypeptide or peptide of interest may additionally comprise transcriptional and translational control regions, which are short arrays of DNA sequences that interact specifically with cellular proteins involved in transcription and translation. These regions include promoters, operator sequences and ribosome binding sites, and are known to those of skill in the art.

It may also be desirable to tag the fusion protein or the secreted fusion protein with at least one tag that may be useful for purifying the secreted fusion protein from the growth medium, for identifying the secreted fusion protein, or for some other purpose. Useful tags include epitope tags that are recognized by a specific antibody, thereby permitting the fusion protein to be purified by affinity chromatography, or to be identified, for example with a fluorescent antibody. Examples of a useful eptiopes are: glutathione-S-transferase, c-myc, poly-histidine, FLAG®, maltose binding protein, influenza A virus haemagglutinin, β-galactosidase and GAL4, or portions thereof. Preferred may be a polyhistidine (poly-His tag). Cognate antibodies that recognize these epitope tags are known to those of skill in the art. For example, polyhistidine is recognized by an anti-His Mab available from Qiagen, FLAG^{®} is recognized by Anti-FLAG M1, M2 and M5 Mabs, available from Sigma/Kodak. Therefore, contemplated herein may be the inclusion, in the recombinant DNA molecule, of at least one nucleotide sequence that encodes a tag amino acid sequence. The tag may be anywhere on the fusion protein, with the possible exception of at the amino-terminal end, or in the leader sequence, of the YebF protein, or a biologically active variant or portion thereof.

It may also be desirable to later cleave the secreted fusion protein, in order to separate the YebF portion (*i.e*., the mature YebF protein) and/or the tag, from the protein, polypeptide or peptide of interest. For example, if the YebF portion and/or tag interferes with the biological activity of the protein, polypeptide or peptide of interest, it may be preferred to cleave either or both of them from the fusion protein. Therefore, contemplated herein may be the inclusion, in the recombinant DNA molecule, of at least one nucleotide sequence that encodes an amino acid sequence, which amino acid sequence facilitates the cleavage of the YebF portion and/or epitope tag and/or the protein, polypeptide or peptide, from the fusion protein. Examples of suitable amino acid sequences for cleavage by specific proteases are: (a) Factor Xa Protease- recognizes the amino acid sequence Ile-Glu-Gly-Arg and cleaves the peptide bond C-terminal of the arginine residue^{24,25}; (b) Tobacco Etch Virus (TEV) NIa protease- recognizes a seven amino acid consensus sequence, Glu-X-X-Tyr-X-Gln/Ser, where X can be various amino acyl residues, and cleaves between the conserved Gln and Ser residues²⁶; (c) PreScission™ Protease- cleaves between the Gln and Gly residues of the recognition sequence of LeuGluValLeuPheGlnlGlyPro^{27,28}; (d) Thrombin - which is used to digest fusion proteins prepared from pGEX vectors (GST Gene fusion) containing the recognition sequence for thrombin²⁹, and (e) Enterokinase - which cleaves after lysine at its cleavage site Asp-Asp-Asp-Asp-Lys, or other basic residues, depending on the conformation of the protein substrate³⁰.

Therefore, a recombinant DNA molecule contemplated herein comprises a nucleotide sequence that encodes the YebF protein and a nucleotide sequence that encodes the protein, polypeptide or peptide of interest, and optionally at least one nucleotide sequence that encodes a tag, and/or optionally at least one nucleotide sequence that encodes a protein cleavage site. These various nucleotide sequences are in frame with one another. Therefore, the fusion protein and secreted fusion protein contemplated herein comprise optionally, at least one tag and/or optionally at least one cleavage site.

The resultant recombinant DNA molecule may then be inserted into an expression vector. Useful vectors for practicing the invention disclosed herein are plasmids, for example pMS119EH, which comprises a lac promoter, and pT7-5, which comprises a T7 promoter. The expression vector selected will depend on which bacterial strain or species is to be used to express the fusion protein.

As is apparent to those of skill in the art, the above sequence of events need not be practiced in the above order, nor need the above methods be used, in order to generate the expression vector comprising the recombinant DNA molecule. For example, the nucleic acid that encodes the protein, polypeptide or peptide of interest may be inserted into the expression vector, followed by the insertion of the nucleic acid that encodes the YebF protein, or vice versa (see Materials and Methods, herein). A nucleic acid that encodes the tag or the protein cleavage site, if used, may be inserted before or after either of the above components of the recombinant DNA molecule are inserted into the expression vector. The expression vector may already comprise a nucleic acid that encodes a tag sequence, obviating the need to add this component separately (see Materials and Methods, herein).

The expression vector comprising the recombinant DNA molecule is then transfected into the appropriate bacterial host, for expression of the fusion protein. Examples of how this can be accomplished are provided examples provided in the Materials and Methods, and Examples herein. As non-limiting examples, the expression vector may be transfected into HB101, BL21 or MG1655. For example, pMS119EH comprising an insert can be used in HB101 or MG1655, and pT7-5 comprising an insert can be used in BL21.

Preferred for use herein may be an expression vector that replicates extrachromosomally, as a greater number of copies of the fusion protein nucleic acid construct can be generated, which will generally result in higher levels of expression and hence secretion.

The present invention contemplates the secretion of many different proteins, polypeptides or peptides of interest. Contemplated herein are peptides as small as 4 amino acids. Also contemplated herein is any protein or polypeptide that can be secreted by the methods disclosed herein, regardless of the size. In the Examples provided, a protein of 48 kD was secreted, suggesting that large proteins can readily be secreted by the method disclosed herein.

Both hydrophilic and hydrophobic proteins may be secreted by the method disclosed herein. In the Examples provided both a hydrophobic protein (hIL2) and a hydrophilic protein (α-amylase or alkaline phosphatase) were secreted.

The bacterial cells are grown in growth medium, such as terrific broth, until such time as is desired to harvest the secreted fusion protein from the medium. The time required depends upon a number of factors relating to the bacterial expression system being used and to the fusion protein being produced. The rate of growth of a particular bacterial strain or species, the rate at which the secreted fusion protein accumulates in the medium, the stability of the secreted fusion protein in the medium, and the time at which bacterial lysis begins to occur (which will contaminate the growth medium) are non-limiting examples of the types of considerations that will affect when the secreted fusion protein is harvested from the culture medium.

Depending upon the level of purity required, which will again depend upon the application for which the secreted fusion protein will be used, the secreted fusion protein may be further purified, for example by affinity chromotography. If desired, the YebF portion and/or the tag may be cleaved from the secreted fusion protein, in order to generate the protein, polypeptide or peptide of interest, alone.

Disclosed herein is the fact that YebF can be used to direct the secretion of proteins, polypeptides and peptides, as fusion proteins, into the medium. The inventors have cloned three different nucleotide sequences of interest into a vector that comprises the nucleotide sequence for *yebF.* Accordingly, disclosed herein is an expression vector that comprises a nucleic acid encoding YebF operatively linked to control nucleic acid sequences needed to initiate, regulate or otherwise direct transcription and the synthesis of YebF. The expression vector may comprise restriction endonuclease sites, or other sites, downstream of the YebF coding region, useful for easy in frame insertion of a nucleic acid encoding a protein, polypeptide or peptide of interest. The expression vector may also comprise a tag nucleotide sequence, as shown in the Materials and Methods herein, or a nucleotide sequence encoding a protein cleavage site.

Also disclosed herein is the fact that the leader sequence of YebF may be used to direct the transport of a protein into the periplasm. Accordingly, disclosed herein is an expression vector that comprises a nucleic acid encoding the leader sequence of YebF operatively linked to control nucleic acid sequences needed to initiate, regulate or otherwise direct transcription and the synthesis of the YebF leader sequence. The expression vector may comprise restriction endonuclease sites, or other sites, downstream of the YebF leader coding region, useful for easy in frame insertion of a nucleic acid encoding a protein, polypeptide or peptide of interest. The expression vector may also comprise a tag nucleotide sequence, as shown in the Materials and Methods herein, or a nucleotide sequence encoding a protein cleavage site. Also disclosed herein is the use of the YebF leader sequence to direct transport of a protein, peptide or polypeptide to the periplasm. Also disclosed herein is a method for expressing a protein, polypeptide or peptide of interest in the periplasm, which method comprises providing an expression vector that encodes a protein comprising the protein, polypeptide or peptide of interest located carboxy-terminally to YebF leader sequence, and expressing the protein in a suitable bacterial cell.

While the invention has been described in conjunction with the disclosed embodiments, it will be understood that the invention is not intended to be limited to these embodiments. On the contrary, the invention is intended to cover alternatives, modifications and equivalents, which may be included within the scope of the invention as defined by the appended claims. Various modifications will remain readily apparent to those skilled in the art. Examples provided above and below are not intended to be limited to those examples alone, but are intended only to illustrate and describe the invention rather than limit the claims that follow.

### EXAMPLES

### Materials and Methods

**Bacterial strains and growth conditions:** The *E. coli* strains used in this study were HB101 (*supE44 hsd20(r_{B}⁻m_{B}⁻) recA13 ara-14 proA2 lacY1 gaIK2 rpsL20 xyl-5 mtl-1*), BL21(DE3)(*hsdS gal(*λ*c*I*ts*857 *ind*1 Sam7 *nin5 lac*UV5-T7 *gene 1*), and MG1655(F- λ-*ilvG-rfb-*50 *rph-*1). Cells were grown at 30°C in Terrific broth (TB). Antibiotics were added at the following concentrations: 100 µg/ml of ampicillin (Amp) and 80 µg/ml chloramphenicol (Cm). Expression of genes under *lac* promoter control was induced with 0.1mM isopropyl-µ-D-thiogalactopyranoside (IPTG).

**Molecular biology techniques and plasmid construction:** DNA manipulation, sequencing and bacterial transformation were essentially as described¹⁷. Expression plasmids were constructed based on pMS119EH¹⁸ containing the *lac* promoter, or pT7-5 with the T7 promoter. PCR amplifications using the touch-down protocol and DNA purification thereafter were carried out as described¹⁹.

The *yebF* gene was amplified from HB101 chromosomal DNA using the following forward and reverse oligomers, respectively, with the indicated restriction sites underlined: yebF5, 5'-GAGAATTCGGAGA-AAAACATGAAAAAAAG-3' containing *Eco*RI (SEQ ID NO: 4); and yebF3, 5'-ATATCTCGAGACGCCGCTGATATTC-3 (*Xho*I) (SEQ ID NO: 5). To tag the gene with 6 histidines at its 3'-end (*yebFH₆*), the amplified DNA, after digestion with *Eco*RI and *Xho*I, was cloned into pCITE-2a(+) (Novagen, US) cut with the same enzymes. The *Eco*RI*-Hind* III fragment carrying *yebFH6* was then subcloned into pMS119EH to generate pYebFH₆/MS, or into pT7-5 to make pYebFH₆/T7.

The mature truncated form of the α-amylase gene (GenBank accession no. AB015592) of *Bacillus subtilis* X-23⁴ was amplified from pAC92 kindly provided by Dr. Spartaco Astolfi-Filho, University of Brasilia, Brazil using the following oligos: amy5, 5'-TAGAATTC**AGGAG**AAAAACATGGTCGACTCGGTCA AAAACGGG-3' (SEQ ID NO: 6) engineered with an *Eco*RI site, a ribosomal binding site (bold), the in-frame initiation codon ATG and *Sal*1; amy3, 5'-ATATCTCGAGATGAGGCGCATT TCC-3 (*Xho*1) (SEQ ID NO: 7). The amplified DNA was cut with *Eco*RI and *Xho*I and replaced the *yebF* fragment (*Eco*RI*-Xho*I) in pYebFH6/MS to create pAmyH₆.

pYebF-AmyH₆ containing the *yebF-amy* fusion gene was created by replacing the *Eco*RI-*Sal*1 fragment in pAmyH₆ with the *Eco*RI*-Xho*I fragment carrying the *yebF* gene from pYebFH₆/MS. Between the *yebF* nucleotide sequence and the amy nucleotide sequence, there is encoded an additional two amino acids- LE- which will link the two proteins.

To fuse the sec-leader sequence of the *yebF* gene to the mature α-amylase gene, the leader sequence was first amplified by PCR using pYebFH₆/MS as the template with Fls (5'-ATATCTCGAGAGCGAAAACTGATGC-3') (SEQ ID NO: 8) containing an *Xho*I restriction site and yebF5 (above) as the oligomers. After digestion with *EcoR*I and *Xho*I, the amplified DNA fragment was inserted into pAmyH₆ to replace the *EcoR*I- *Sal*I sequence, which generated pLS-AmyH₆. The sequence of this hybrid gene was verified by sequencing.

To construct pYebE-hIL2H₆, the hIL-2 gene was amplified by PCR from pBM806, a plasmid containing an hIL-2 gene provided by Biomira Inc. (Edmonton, Canada) using the following oligonucleotides: hil5 with *Sal*I, GATCATGTCGACGCTCCGACCTCCAGC (SEQ ID NO: 9)and hil3 with Xhol, ATATCTCGAGGGTCAGGGTGGAGAT (SEQ ID NO: 10). The *Sal*I*-Xho*I fragment of the PCR product was inserted into *Xho*I site in pYebFH6/MS to generate pYebF-hIL2H₆. Between the *yebF* nucleotide sequence and the *hIL-2* nucleotide sequence, there is encoded an additional two amino acids- LE- which will link the two proteins, which is then followed by the six histidine residues as the tag.

The amino acid sequence of mature hIL-2 (*i.e*., lacking the 20 amino acid leader sequence) is:

To make pYebF-phoAH₆/T7 plasmid, the *pho*A gene was PCR-amplified with the following oligos: phoA5 with *Xho*I*,* GCATATCTCGAGCGGACACCAGAAATGCC (SEQ ID NO: 12); and phoA3 with *Sal*I, CGATAGTCGACTTTCAGCCCCAGAGC (SEQ ID NO: 13). The *Xho*I*-Sal*I fragment of the PCR product was inserted into *Xho*I site in pYebFH₆/T7 to generate pYebF-phoAH₆/T7.

**Protein expression and treatment of samples:** A single colony from LB plates or a small aliquot of cold cell suspension stored in 40% glycerol at -20°C was inoculated into 1.5 ml of TB and grown overnight at 30°C. After harvesting by centrifugation the cells were washed once in 1.5 ml of fresh TB, and were suspended in 1.5 ml of fresh TB and inoculated at 1% by volume into fresh TB for expression studies. Protein expression was induced by addition of 0.1mM IPTG or 0.5 mM IPTG at 0.6 OD₆₀₀. Cells were harvested at the indicated time points in the experiments. After harvesting by centrifugation at room temperature to avoid cold shock, cell pellets were washed once with a volume equal to the original culture volume of 100 mM MOPS (pH7.0). The resulting medium supernatant was filtered using a 0.22 µm Millipore filter to remove un-pelleted cells.

**Subcellular fractionation:** Periplasm was separated from cytoplasm by the osmotic shock method.²¹

**α-amylase activity assay:** This assay is based on the 3,5-dinitrosalicylic acid method as described by *Ohdan et al.*⁸ using starch as the substrate for the enzyme. The reaction was stopped by adding 200 µl of DNSA in 0.4N NaOH to 200 µl of the reaction mixture after 10 minutes incubation at 58°C with shaking once per minute. The mixture was boiled for 5 minutes thereafter, diluted with 1 ml of water, and spun for 1 minute to pellet starch. Finally, readings were taken at 540nm. The enzyme activity was expressed in moles of glucose released per minute that is equivalent to the amount of reducing sugar released by the enzyme from starch in the reaction. A pair of every sample to be assayed was set up. One is for the reaction, the other for background reading, to which 200 µl of DNSA was added before the reaction starts.

To measure the activity in the medium or in the osmotic shock fluid, the liquid was directly added to the reaction mixture. To assay the activity in cells or in the resulting spheroplasts from the osmotic shock, the cell suspension or the spheroplast suspension in 10mM Tris-HCI (pH7.0) was mixed with equal volume of lysis buffer (pH 8.0) consisting of 70 µg/ml lysozyme, 6 mM EDTA, 2% Triton X-100 to break cells or spheroplasts. After lysis was complete on ice, 1 µl of 210 mM CaCl₂ was added to every 70 µl of the lysate to overcome the EDTA and allow for 0.1mM Ca²⁺ in the reaction mixture as required for α-amylase activity.

**Alkaline phosphatase activity assay:** 1 ml of the reaction consists of 100mM Tris-HCl (pH8.0), 10mM MgCl₂, 2mM ZnCl₂ and 0.04% pNPP. The reaction after incubation of indicated period was stopped by addition of 40 µl of 5M NaOH followed by 1 minute of centrifugation at 14,900rpm before taking readings at 410nm. To measure the background readings, samples were added to the mixtures after the reactions were stopped by NaOH. To calculate the enzyme activities, ε₄₁₀ = 17,800 M⁻¹ cm⁻¹ for p-nitrophenolate anion (pNP), the yellow product released from pNPP.

**Purification of YebFH₆ and protein chemistry:** Total protein in the culture medium of *E. coli* HB101/pYebFH₆/MS grown overnight (15 hr) after induction with IPTG was precipitated by ammonium sulfate at 70% saturation at 4°C in the presence of 0.2 mM PMSF. The pellet was dissolved in 20 mM Tris-HCI (pH7.5) containing 0.2mM PMSF, 10 mM imidazol and 500 mM NaCl. Purification of YebFH₆ was achieved using a nickel affinity column (Amersham). This partially purified protein was further purified using a reversed-phase HPLC on a 300Å C8 column (1mm x 15 cm) eluted with a gradient of acetonitrile from 0% to 2% in the presence of 0.05% TFA at the flow rate of 100 µl/min. A single major peak was observed at the 25th min. This peak fraction was collected and used for both MALDI-TOF linear mode mass spectrometry (Voyager 6064, Applied Biosystems) and N-terminal amino acid sequence determination using Routine 3.0 on a Hewlett Packard G1000A Protein Sequencer.

**Protein electrophoresis and Western blotting:** The protein samples from either the culture medium or the cells were prepared by precipitation with 5% trichloroacetic acid (TCA). After centrifugation in an Eppendorf tube at 14,900 rpm at room temperature for 5 minutes, the pellet was washed once by suspending it in 1 ml of water and centrifuging for 5 minutes to remove residual TCA. To almost completely extract TCA from the pellets, 1.5 ml of -20°C cold acetone was added to each tube. The tube was then vortexed and kept at -20°C for 20 minutes before centrifugation as above. The pellet was retained and subjected to one additional acetone wash. The final pellet was dissolved in 50 mM Tris-HCl, pH 8.0 before adding an equal volume of electrophoresis loading buffer. Proteins were separated on 8.5% SDS-Tricine gel²² and then transferred to a nitrocellulose membrane for immunoblotting. To detect the histidine tag, monoclonal mouse antibody against histidine tag (Qiagen) was used. CRP was detected using a rabbit polyclonal antiserum against CRP, kindly provided by Dr.Hiroji Aiba, Nagoya University, Japan. Immunoblots were developed by enhanced chemiluminescence using the ECL kit (Amersham).

**IL-2 bioassay:** The biological assay of YebF-hIL2 was based on the Lei *et al.* method²³ using the IL-2 dependent T lymphocyte cell line CTLL-2 with modifications. Prior to the assay, the YebF-hIL2 sample was prepared by passing the *E. coli* culture medium harvested 3 hours after induction with 0.1mM IPTG from either HB 101/pYebF-hIL2H₆ or pMS119EH (control) through a 0.22 µm Millipore filter. The cell culture medium (RHFM) for CTLL-2 uses RPMI 1640 supplemented with 10% (v/v) fetal calf serum, 0.1 mM β-mercaptoethanol, 25mM HEPES pH7.5, 2 mM L-glutamine. 50 µl of fresh CTLL-2 cells (10⁶ cells/ml) in RHFM were placed in a well of a 96-well plate. 50 µl of standard hIL-2 (kindly provided by Dr. C. Bleackley, University of Alberta) or YebF-hIL2 samples diluted with RHFM was added. The cells were incubated at 37°C with 5% CO₂ for 20 hours. Cell proliferation was measured using the MTT (*3-(4,5-dimethylthiazol-2-yl)-2,5-diphenyl tetrazolium bromide*) assay. 5 mg of MTT/ml was prepared in phosphate buffered saline and sterilized. 10 µl of MTT was added to each well followed by 4 hours incubation at 37°C. 150 µl of isopropanol containing 0.04N HCl was added to each well and mixed thoroughly with a pipettor to dissolve the resulting crystals. The absorbance was read at 560 nm. Each value is the average of three assays.

### Characterization of the E. coli YebF leader

Examination of the DNA sequence of the *yebGFE* operon revealed a typical ribosome-binding site (GGAG, Blattner coordinates 1928421 - 1928424 in MG1655) upstream from the second ATG (1928412 - 1928414) in the region between *yebG* and *yebF.* Although the first ATG (Blattner coordinates 1928424 - 1928426) could initiate in-frame translation of YebF, and this is the way it is reported in databases such as NCBI (B1847) and Swiss-Prot (P33219), no ribosome-binding site could be found preceding this ATG. The *yebF* gene was cloned in the expression vector pMS119EH under *lac* promoter control starting from nucleotide G (Blattner coordinate 1928424) without addition of a ribosome binding site and with the addition of a 6-histidine tag at the carboxyl terminus. The His-tagged YebF protein was positively expressed as shown by immunoblotting (see below).

The N-terminal amino acid sequence of *E. coli* YebF is as follows:
(-21)MKKRGAFLGLLLVSACASVFA(-1) (1)ANNE....

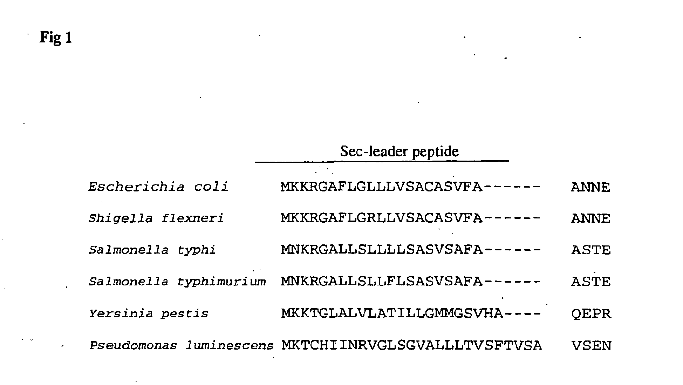

This sequence resembles the motif characteristic of lipoproteins⁴, including the fatty acid-acylation site (Cys-6). This motif is also conserved in YebF from *Shigella flexneri* (*97%* identity). However, this Cys is missing in other similar proteins such as STY2087 from *Salmonella typhi* (77% identity), YebF from *Salmonella typhimurium* (76% identity), YP01779 from *Yersinia pestis* (43% identity) and PLU2692 from *Photorhabdus luminescens* (40% identity). Therefore, YebF may not be a lipoprotein as suggested in several databases (B 1847 at NCBI and P33219 at Swiss-Prot). The amino terminal sequence of the *E. coli* YebF protein also contains a typical 21 amino acid *sec-*leader peptide including 3 basic residues (-20 Lys-Lys-Arg-18) at the N-terminal end, followed by a 16 residue long hydrophobic core and a small residue (Ala-1) which could serve as the leader peptidase I cleavage site (Fig.1). A similar leader sequence was found in all YebF related proteins (Fig.1) suggesting that *E. coli* YebF may be secreted to the periplasm.

### YebF localization

To determine where YebF was localized, a 200 ml culture of HB101/pYebFH₆/MS was grown in TB at 30°C until the optical density at 600 nm reached 0.6. The culture was induced with 0.1 mM IPTG and harvested 4 hours after induction and washed once at room temperature with 200 ml of 100 mM MOPS (pH7.0). Cells were subjected to cold osmotic shock (Neu and Heppel, 1965) to separate periplasm from the cytoplasm. Protein samples from these resulting fractions and immunoblotting were prepared as described in Materials and Methods.

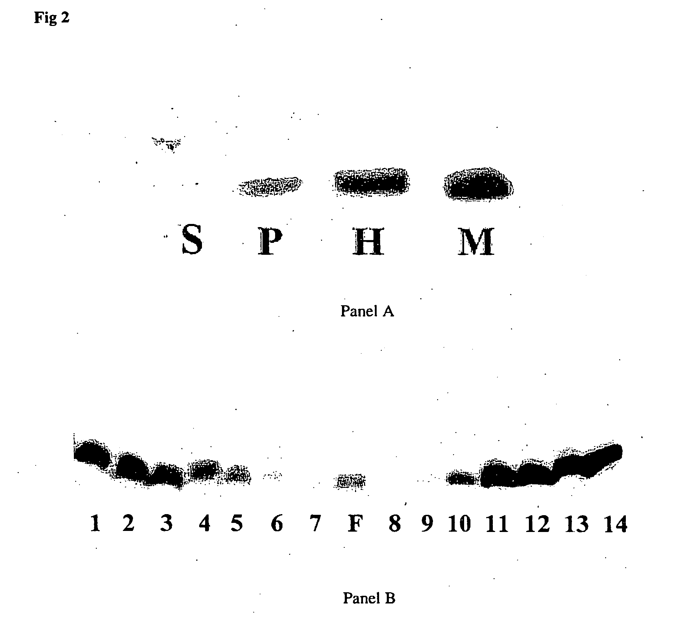

YebF was located in the periplasm and surprisingly also in the culture medium. No YebF was detected in the spheroplasts after cold osmotic shock (Fig. 2A).

To determine the effect of inhibition of protein synthesis on secretion, 10 ml HB101/pYebFH₆/MS cells were grown as described for Fig. 2A. After harvesting, cells were washed once with 35 ml of fresh TB and suspended in 10 ml of fresh TB containing 80 µg chloramphenicol/ml. 1.5 ml of cells were taken at the time points indicated in Fig. 2B. Cells and medium were separated by centrifugation at room temperature to avoid cold shock. Immunoblotting was conducted as described in the Materials and Methods using a monoclonal antibody directed against the His₆ tag.

When protein synthesis was inhibited, the level of YebF in the periplasm gradually declined to a trace level while it progressively accumulated in the medium during the subsequent incubation period (Fig. 2B).

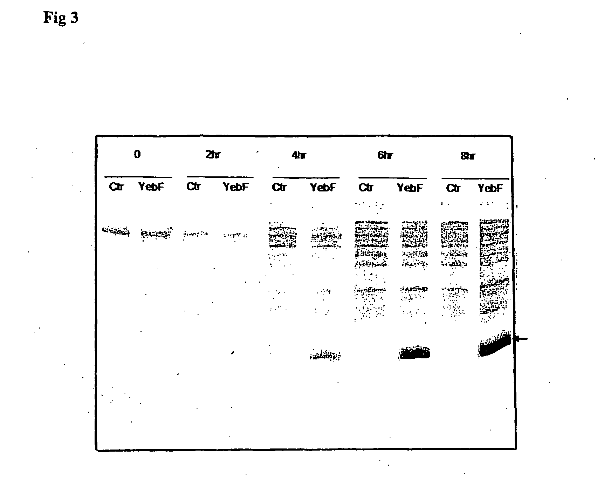

To demonstrate that YebF protein accumulates in the medium during growth, cells were induced with 0.1 mM IPTG, grown overnight, collected at the indicated time after induction and prepared as described in the Materials and Methods. As can be seen in Fig. 3, all the YebF was located in the culture medium.

When cells were induced and grown overnight, all the YebF was located in the medium (data not shown). Taken together, this data suggested that YebF is most likely an extracellular, soluble protein (see below).

### Characteristics of the secreted YebF

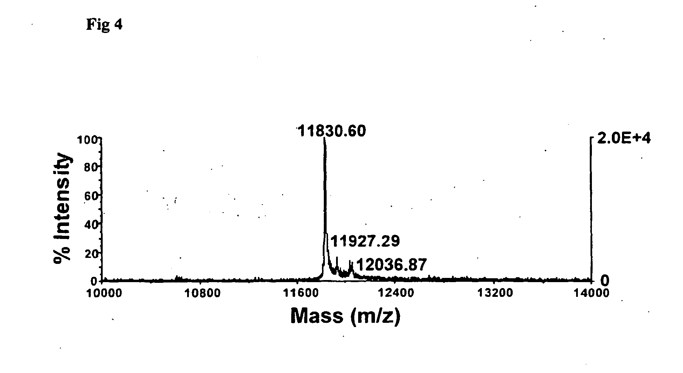

The secreted YebF-His₆ in both the periplasm and culture medium migrated on SDS-Tricine polyacrylamide gels at a molecular weight corresponding to the mature His-tagged protein (~12 kD) (Fig. 3). YebF-His₆ was purified from the culture medium as described in the Materials and Methods and N-terminal amino acid sequence analysis revealed the sequence to be Ala-Asn-Asn-Glu-Thr-Ser-Lys-Ser-Val-Thr. This result indicates that YebF is cleaved immediately after the 21-amino acid sec-leader and not preceding the Cys at position -6 (Fig. 1). To examine whether there were any fatty acids bound to the mature YebF protein, the mass was determined to be 11,829.2+/-1.4 Daltons by Maldi-TOF linear mode mass spectrometry (Fig. 4). The calculated mass of mature His-tagged YebF with the 6-histidine tag and Leu-Glu linking amino acids, was 11,860.3 Daltons. This is 31.1 Daltons (0.26%) larger than the experimentally determined mass. If there were any covalently attached fatty chains, the mass determined by mass spectrometry would be expected to be larger than the calculated mass, suggesting therefore that YebF is not a lipoprotein.

### Secretion of YebF into the medium

The presence of YebF in the culture medium could be a result of cell lysis, leakage through the outer membrane, a natural cell secretion process or some combination. To test for lysis, immunoblotting was used to compare the subcellular localization of YebF and catabolite repressor protein (CRP), a well-known DNA binding protein residing in the cytoplasm. *E. coli* HB101/pYebFH₆/MS was grown in 50 ml of TB at 30°C until OD₆₀₀ reached 0.6 and induced with 0.1 mM IPTG. Ten ml of cell culture was harvested at 2, 4 and 6 hours after induction. The cells were washed once in 10 ml of 100 mM MOPS and suspended in 10 ml of the same MOPS buffer. The medium was filtered through a 0.22 µm Millipore filter. Both cell suspension and medium were mixed with 2.5 ml of 25% trichloroacetic acid (TCA) to precipitate proteins. TCA trapped in the pellets was removed by two extractions using -20°C acetone. Protein samples were loaded onto two parallel gels which were subsequently subjected to immunoblotting.

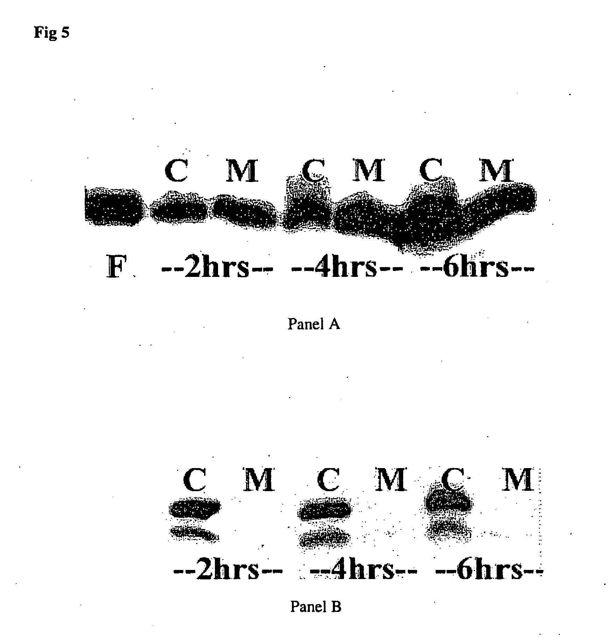

In cells, both CRP and YebF were readily detected. In the medium, YebF was detected at all experimental time points (Fig. 5A). CRP, however, was not observed in the medium at 2 and 4 hours after induction (Fig. 5B). A trace amount of CRP was detected at 6 hours, which indicates the beginning of inevitable cell lysis (Fig. 5). This indicates that the YebF found in the medium up to 4 hours post induction did not result from cell lysis.

In order to investigate the possible involvement of outer membrane leakage, two chimeric genes were constructed in the vector pMS119EH, under lac-promoter control, by fusing the leaderless, mature α-amylase gene from *Bacillus subtilis*⁹ to either the 3' end of the *yebF* gene (pYebF-AmyH₆) or directly to the sec-leader sequence of *yebF* (pLS-AmyH₆). In addition, the leaderless mature α-amylase gene in the same vector (pAmyH₆) was used as a control. The mature α-amylase without any signal sequence should remain in the cytoplasm. Cells were grown in HB101 and induced as in Fig. 2. Cells were harvested 4 hours after induction with 0.1mM IPTG, prior to any cell lysis and subjected to cold osmotic shock²¹ and α-amylase activity quantitated as described in Materials and Methods.

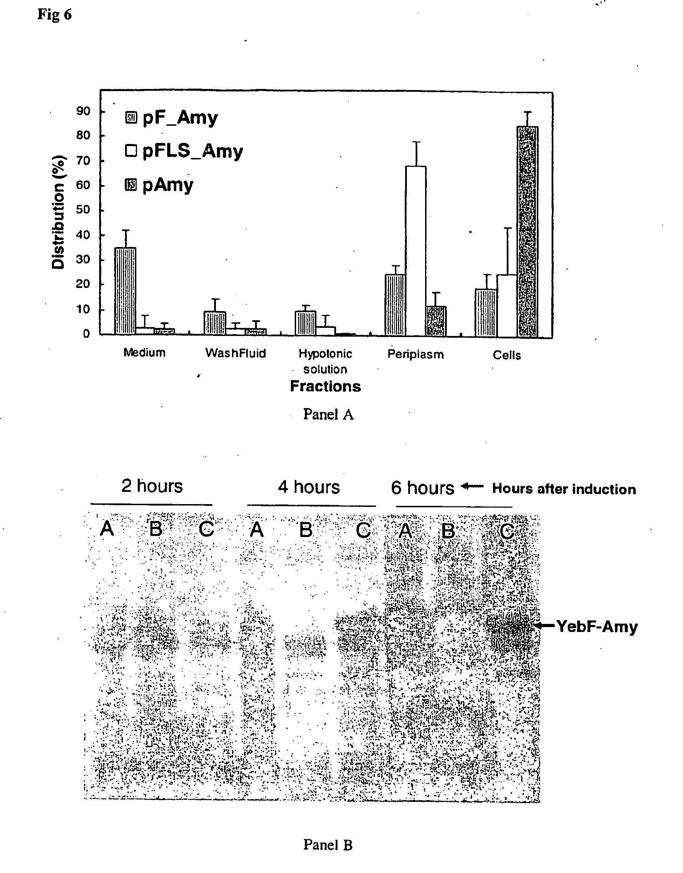

HB101 cells with pMS119EH produced an undetectable level of α-amylase either in the medium or in the cells (data not shown). Only α-amylase fused to full-length YebF could be found in the medium with 35% of the total activity in the medium. The leaderless α-amylase and α-amylase fused to the leader peptide of YebF were expressed but remained inside cells, and less than 5% of each on average were detected in the medium (Fig.6A). These data were confirmed by the protein profile in the medium shown in Fig. 6B.

Using the cold osmotic shock method, the periplasm of cells was separated from the cytoplasm. As shown in Fig.6A, over 68% of the α-amylase fused to the leader of YebF was recovered in the periplasm. 85% of leaderless α-amylase remained in the cytoplasm. 12 % of the leaderless α-amylase found in the periplasm could be due to cell breakage during the cold osmotic shock. These data demonstrated that the YebF leader was able to direct the fusion protein from the cytoplasm to the periplasm using the sec-dependent translocation system but could not release the protein to the medium. Therefore these data together with the absence of α-amylase in the medium from cells harboring plasmid pLS-AmyH₆ indicate that the outer membrane was able to prevent mature α-amylase (the YebF leader peptide was removed by leader peptidase I) from leaking out of cells during normal growth.

The YebF-α-amylase fusion protein was able to cross the outer membrane from the periplasm (Fig 6A). This demonstrates that YebF played an essential role in targeting the fusion protein for secretion across the outer membrane to the medium. In addition, Fig. 2 shows no apparent difference in the mobility of YebF protein in the periplasm or in the culture medium. This indicates that no additional cleavage occurred during secretion across outer membrane. This is also supported by the coincidence of the molecular weight calculated from the sequence (11,860 dalton) with that determined by mass spectrometry (11,829 dalton).

To further demonstrate that the presence of YebF in the medium is due to secretion from cells, rather than periplasmic leakage, the release of YebF-α-amylase was compared to the release of alkaline phosphatase, an endogenous well-defined periplasmic enzyme, in GZ39T/pYebF-AmyH₆ cells. Because alkaline phosphatase is induced by phosphate-starvation, it is very poorly expressed in wild-type *E. coli* cells grown in rich medium containing inorganic phosphate salts. To overcome this, a derivative of TG1, GZ39T, that expresses about 10 times more alkaline phosphatase than TG1 in rich medium, was isolated.

GZ39T/pYebF-AmyH₆ was grown in 25 ml of TB at 30°C. Cells were harvested at 7 hours after induction with 0.05 mM IPTG, washed with 100 mM MOPS (pH7.0) and suspended in 1.5 ml of 30 mM Tris-HCl (pH8.0). 200 µl of the cells were mixed with 300 µl of cell lysis buffer (see Methods and Materials). This cell lysate was used for both α-amylase and alkaline phosphatase activity assays. To measure α-amylase activity in the medium, 35 µl of the medium was directly used. To measure alkaline phosphatase activity, 8 ml of the medium was filtered three times through an Amicon ultracentrifugal filter (MWCO 30KD) with 50 mM MOPS (pH7.0) to reduce phosphate and colored substances and finally concentrated to 0.6 ml. 200 µl of this preparation was used for the assay (see Methods and Materials). Alkaline phosphatase was assayed at 37°C for 60 minutes in the dark. The results are shown in Fig. 7A, which shows that 42.6% of YebF-α-amylase was secreted to the medium compared to only 5.6% of alkaline phosphatase.

The possibility that over-expression of YebF causes destablization of the outer membrane was investigated by testing the permeability of 5 antibiotics: erythromycin, rifampicin, vancomycin, bacitracin and azithromycin. Little change in MICs (minimum inhibitory concentrations) was observed for cells expressing YebF or YebF-α-amylase in response to these agents, compared to cells either without plasmid or with pMS119EH (Fig. 7B).

### Secretion capability of different E. coli strains

3 ml of cells harboring pYebFH₆/MS or pYebFH₆/T7 were grown and induced as in Fig.2A. Protein sample preparation and immunoblotting were conducted as described in the Materials and Methods. Monoclonal antibody against the histidine tag was used to monitor YebF-His₆.

MG1655 (a K12 strain), BL21(DE3) (a B strain) and HB101 (a hybrid of K12 and B) all secrete YebF into the medium, although the efficiency varied (Fig. 8). This result may suggest that *E. coli* cells including common laboratory strains still possess the capability of secreting proteins including YebF to the surrounding milieu.

### Recombinant protein production with YebF

Fusion protein gene constructs of leaderless α-amylase of *B. subtilis* X-23⁸ (48kD), leaderless *E. coli* alkaline phosphatase (94 kD as a dimer) and the medically relevant protein human interleukin-2 (hIL-2) (15 kD) to the carboxy terminus of YebF were made as described in the Materials and Methods. These represent both hydrophilic (α-amylase and alkaline phosphatase) and hydrophobic (hIL-2) proteins. All three passenger proteins carried a His₆ tag at their C-terminus.

Cells comprising these fusion protein gene constructs were grown in 25 ml of TB at 30°C. 10 mM DTT was added to one culture at the same time as 0.05 mM IPTG was added to induce expression. Samples were taken 3 hours after induction.

Fig. 9A shows the expression of mature YebF-hIL2 fusion protein in HB101/pYebF-hIL2H₆. The fusion protein was secreted to the medium at 3 hours after induction under regular growth conditions. With the presence of 10 mM DTT in the medium, however, cells did not secrete the fusion protein to the medium, although comparable expression inside cells was seen. Activity assays for the YebF-hIL-2 fusion protein based on the growth of CTLL-2 T-lymphocytes *in vitro* showed the secreted protein in the medium was active (Fig. 9B). The activity of the fusion protein was 43,800 units of hIL-2/ml of medium.

Fig. 10 shows that by 9 hours after induction 75% of mature YebF-α-amylase fusion protein was secreted from cells. It also appears that the fusion protein was stable in the medium and the maximal secretion of the YebF-α-amylase occurred after the culture reached stationary phase. From a 1-liter scale experiment, 31 mg of the purified YebF-α-amylase fusion protein with a specific activity of 380.7 µmol Glucose/min/mg protein was obtained, using nickel affinity chromatography. The specific activity of α-amylase in the initial culture medium varies with the time after induction from 150 mol Glucose/min/mg protein at 4 hours to 85 mole Glucose/min/mg protein at 9 hours after induction. It is 3.4 µmole Glucose/min/mg protein from *B. subtilis* culture⁴. The high specific activity here is also reflected in the simple protein profile of the medium shown in Fig. 6B.

Mature YebF-alkaline phosphatase fusion protein was also expressed from pYebF-phoAH₆/T7 plasmid in BL21(DE3) cells under the control of the T7 promoter. 23% was secreted to the medium at 7 hours after induction with IPTG.

### REFERENCES

The following references are cited in the application as numbers in brackets ([]) at the relevant portion of the application.
1. Lomba MR, et al. Identification of yebG as a DNA damage-inducible Escherichia coli gene. FEMS Microbiol. Lett. 156: 119-122 (1997).
2. Courcelle J, et al. Comparative gene expression profiles following UV exposure in wild-type and SOS-deficient Escherichia coli. Genetics. 158: 41-64 (2001).
3. van Dyk TK, et al. A genomic approach to gene fusion technology. Proc. Natl. Acad. Sci. USA. 98: 2555 - 2560 (2001).
4. Brokx SJ, et al. Genome-Wide Analysis of Lipoprotein Expression in Escherichia coli MG1655. J. Bacteriol. 186: 3254 - 3258 (2004).
5. Shokri A, et al. Cell and process design for targeting of recombinant protein into the culture medium of Escherichia coli. Appl. Microbiol. Biotechnol. 60: 654-664 (2003).
6. Pugsley AP and Francetic O Protein secretion in Escherichia coli K-12: dead or alive? Cell Mol. Life Sci. 54: 347-52 (1998).
7. Errington J and Mountain A Is Bacillus an alternative expression system? In: Protein production by biotechnology. Edited by Harris TJR. Elsevier, pp.1-15.(1990).
8. Ohdan K et al. Characteristics of two forms of alpha-amylases and structural implication. Appl. Environ. Microbiol. 65: 4652-4658 (1999).
9. Poquet I et al. Stable periplasmic secretion intermediate in the general secretory pathway of Escherichia coli. EMBO. J. 12: 271-278 (1993).
10. He SY et al. Cloned Erwinia chrysanthemi out genes enable Escherichia coli to selectively secrete a diverse family of heterologous proteins to its milieu. Proc. Natl. Acad. Sci. USA. 88: 1079-1083 (1991).
11. Francetic O, et al. Expression of the endogenous type II secretion pathway in Escherichia coli leads to chitinase secretion. EMBO. J. 19: 6697-703 (2000).
12. Weiner JH, et al. A novel and ubiquitous system for membrane targeting and secretion of cofactor-containing proteins. Cell. 93:93-101 (1998).
13. Stathopoulos C, et al. Secretion of virulence determinants by the general secretory pathway in gram-negative pathogens: an evolving story. Microbes. Infect. 2: 1061-1072 (2000).
14. Francetic O and Pugsley AP The cryptic general secretory pathway (gsp) operon of Escherichia coli K-12 encodes functional proteins. J. Bacteriol. 178: 3544-3549 (1996).
15. Robb RJ, Interleukin 2: the molecule and its function. Immunol. Today 5:203-209 (1984).
16. Masui Y, et al. Microheterogeneity of recombinant products: human interleukin 1α and 1β. Pp167-172. In: Curr. Commun. Mol. Biol. - Therapeutic peptides and proteins. Edited by Marshak D and Liu D. Cold Spring Harbor Laboratory Press, New York (1989).
17. Sambrook J and Russell DW Molecular cloning, a laboratory manual. 3rd ed. Cold Spring Harbor Laboratory Press, Cold Spring Harbor, New York (2001).
18. Strack B, et al. A common sequence Motif, -E-G-Y-A-T-A-, identified within the primase domains of plasmid-encoded I- and P-type DNA primases and the α protein of the Escherichia coli satellite phage P4. J. Biol. Chem. 267: 13062-13072 (1992).
19. Zhang G and Weiner JH CTAB-mediated purification of PCR products. Biotechniques 29: 982-986 (2000).
20. Barros E, et al., A novel cloning system for direct screening using a suicidal strategy. Gene. 179: 287-289 (1996).
21. Neu, HC and Heppel LA, The release of enzymes from Escherichia coli by osmotic shock and during the formation of spheroplasts. J. Biol. Chem. 240:3685-3692 (1965).
22. Schagger H and von Jagow G Tricine-sodium dodecyl sulfate-polyacrylamide gel electrophoresis for the separation of proteins in the range from 1 to 100 kDa. Anal. Biochem. 166: 368-379 (1987).
23. Lei H, et al. Induction of potent antitumor response by vaccination with tumor lysate-pulsed macrophages engineered to secrete macrophage colony-stimulating factor and interferon-g. Gene Ther. 7: 707-713 (2000).
24. Aurell L, et al. A New and Highly Specific Chromogenic Substrate for Factor Xa. Throm. Res. 11:595-6 09 (1977).
25. Ellinger S, et al. Cleavage of Procaryotically Expressed Human Immunodeficiency Virus Fusion Proteins by Factor Xa and Application in Western Blot (Immunoblot) Assays. J. Clin. Microbiol. 27:971-9 76 (1989).
26. Carrington JC and WG Dougherty A viral cleavage site cassette: identification of amino acid sequences required for tobacco etch virus polyprotein processing. Proc. Natl. Acad. Sci. USA 85: 3391-3395 (1988).
27. Walker PA et al. Efficient and rapid affinity purification of proteins using recombinant fusion proteases. Bio/Technology 12(6):601-5 (1994).
28. Cordingley MG. et al. Substrate requirements of human rhinovirus 3C protease for peptide cleavage in vitro. J. Biol. Chem. 265(16): 9062-5 (1990).
29. Chang JY et al. Thrombin specificity. Selective cleavage of antibody light chains at the joints of variable with joining regions and joining with constant regions. Eur. J. Biochem. 151(2): 225-30 (1985).
30. Collins-Racie, LA et al. Production of recombinant bovine enterokinase catalytic subunit in Escherichia coli using the novel secretory fusion partner DsbA. Biotechnology, 13(9): 982-7 (1995).
31. Adelman JP et al. In vitro deletional mutagenesis for bacterial production of the 20,000-dalton form of human pituitary growth hormone. DNA 2(3):183-93 (1983).
32. Viera et al. Methods Enzymol. 153: 3 (1987)
33. Ausubel, Current Protocols in Molecular Biology, Eds. Ausubel et al. (John Wiley & Sons Inc NY) (1995-1999).
34. Zaccolo M et al., An approach to random mutagenesis of DNA using mixtures of triphosphate derivatives of nucleoside analogues. J. Mol. Biol. 255(4):589-603 (1996).
35. Stemmer WP DNA shuffling by random fragmentation and reassembly: in vitro recombination for molecular evolution Proc. Natl. Acad. Sci. USA 91(22):10747-51(1994).
36. Shafikhani S et al. Generation of large libraries of random mutants in Bacillus subtilis by PCR-based plasmid multimerization. Biotechniques 23(2):304-10 (1997).
37. Korpela T. *et al.* Microbial protein expression system. International Publication No. WO 00/66756, published November 9, 2000.
38. Batisson I et al. Full capacity of recombinant Escherichia coli heat-stable enterotoxin fusion proteins for extracellular secretion, antigenicity, disulfide bond formation, and activity. Infection and Immunity, American Society for Microbiology. Washington. 68(7):4064-74 (2000-07-01).

## Claims

1. A method of producing a protein, polypeptide or peptide of interest in a suitable bacterial cell that comprises:
(a) providing an expression vector that encodes a fusion protein comprising YebF including the leader sequence to direct the protein, polypeptide or peptide of interest to the periplasm, fused amino-terminally to the protein, polypeptide or peptide of interest, and
(b) expressing the fusion protein in the bacterial cell to thereby generate a secreted fusion protein.

2. The method of claim 1, further comprising the step of purifying the secreted fusion protein from the medium in which the bacterial cell is growing.

3. The method of claim 1 or 2, wherein the bacterial cell is *Escherichia coli.*

4. The method of claim 1, 2 or 3, wherein the fusion protein further comprises at least one tag.

5. The method of claim 1, 2, 3 or 4, wherein the fusion protein further comprises at least one protein cleavage site.

6. The use of YebF to direct the secretion of a protein, polypeptide or peptide of interest into bacterial growth medium, wherein the YebF is fused amino-terminally to the protein, polypeptide or peptide of interest.

7. The use of the leader sequence of YebF to direct transport of a protein, peptide or polypeptide of interest to the periplasm, wherein the leader sequence of YebF is fused amino-terminally to the protein, polypeptide or peptide of interest.

8. A method for expressing a protein, polypeptide or peptide of interest in the periplasm, which method comprises:
(a) providing an expression vector that encodes a fusion protein comprising the protein, polypeptide or peptide of interest located carboxy-terminally to the leader sequence of YebF, and
(b) expressing the protein in a suitable bacterial cell.

9. The method of claim 8, wherein the expression vector is operatively linked to control nucleotide sequences that direct the transcription and the synthesis of the leader sequence of YebF.

10. The method of claim 9, wherein the nucleotide sequence that encodes the protein, polypeptide or peptide of interest is located downstream of the nucleotide sequence that encodes the leader sequence of YebF.

11. The method of claim 10, wherein the fusion protein further comprises at least one tag.

12. The method of claim 10, wherein the fusion protein further comprises at least one protein cleavage site.

13. The method of claim 8, wherein the bacterial cell is *Escherichia coli.*

## Patentansprüche

1. Ein Verfahren zum Herstellen eines Proteins, Polypeptids oder Peptids von Interesse in einer geeigneten Bakterienzelle, das Folgendes beinhaltet:
(a) Bereitstellen eines Expressionsvektors, der ein Fusionsprotein codiert, welches YebF einschließlich der Leitsequenz, um das Protein, Polypeptid oder Peptid von Interesse zum Periplasma zu lenken, aminoterminal mit dem Protein, Polypeptid oder Peptid von Interesse fusioniert beinhaltet, und
(b) Exprimieren des Fusionsproteins in der Bakterienzelle, um dadurch ein abgesondertes Fusionsprotein zu erzeugen.

2. Verfahren gemäß Anspruch 1, das ferner den Schritt des Reinigens des abgesonderten Fusionsproteins aus dem Medium, in dem die Bakterienzelle wächst, beinhaltet.

3. Verfahren gemäß Anspruch 1 oder 2, wobei die Bakterienzelle *Escherichia coli* ist.

4. Verfahren gemäß Anspruch 1, 2 oder 3, wobei das Fusionsprotein ferner mindestens eine Markierung beinhaltet.

5. Verfahren gemäß Anspruch 1, 2, 3 oder 4, wobei das Fusionsprotein ferner mindestens eine Proteinspaltungsstelle beinhaltet.

6. Verwendung von YebF, um die Absonderung eines Proteins, Polypeptids oder Peptids von Interesse in ein Bakterienwachstumsmedium zu lenken, wobei das YebF aminoterminal mit dem Protein, Polypeptid oder Peptid von Interesse fusioniert ist.

7. Verwendung der Leitsequenz von YebF, um den Transport eines Proteins, Peptids oder Polypeptids von Interesse zum Periplasma zu lenken, wobei die Leitsequenz des YebF aminoterminal mit dem Protein, Polypeptid oder Peptid von Interesse fusioniert ist.

8. Ein Verfahren zum Exprimieren eines Proteins, Polypeptids oder Peptids von Interesse im Periplasma, wobei das Verfahren Folgendes beinhaltet:
(a) Bereitstellen eines Expressionsvektors, der ein Fusionsprotein codiert, das das Protein, Polypeptid oder Peptid von Interesse, welches carboxyterminal zu der Leitsequenz von YebF angeordnet ist, beinhaltet, und
(b) Exprimieren des Proteins in einer geeigneten Bakterienzelle.

9. Verfahren gemäß Anspruch 8, wobei der Expressionsvektor funktionell mit Kontrollnukleotidsequenzen verknüpft ist, die die Transkription und die Synthese der Leitsequenz von YebF lenken.

10. Verfahren gemäß Anspruch 9, wobei die Nukleotidsequenz, die das Protein, Polypeptid oder Peptid von Interesse codiert, nach der Nukleotidsequenz, die die Leitsequenz von YebF codiert, gelegen angeordnet ist.

11. Verfahren gemäß Anspruch 10, wobei das Fusionsprotein ferner mindestens eine Markierung beinhaltet.

12. Verfahren gemäß Anspruch 10, wobei das Fusionsprotein ferner mindestens eine Proteinspaltungsstelle beinhaltet.

13. Verfahren gemäß Anspruch 8, wobei die Bakterienzelle *Escherichia coli* ist.

## Revendications

1. Une méthode pour produire une protéine, un polypeptide ou un peptide d'intérêt dans une cellule bactérienne adéquate qui comprend :
(a) fournir un vecteur d'expression qui code pour une protéine de fusion comprenant YebF incluant la séquence de tête pour diriger la protéine, le polypeptide ou le peptide d'intérêt vers le périplasme, fusionné de façon amino-terminale à la protéine, au polypeptide ou au peptide d'intérêt, et
(b) exprimer la protéine de fusion dans la cellule bactérienne pour générer de ce fait une protéine de fusion sécrétée.

2. La méthode de la revendication 1, comprenant de plus l'étape consistant à purifier la protéine de fusion sécrétée dans le milieu dans lequel la cellule bactérienne est en croissance.

3. La méthode de la revendication 1 ou la revendication 2, dans laquelle la cellule bactérienne est *Escherichia coli.*

4. La méthode de la revendication 1, la revendication 2 ou la revendication 3, dans laquelle la protéine de fusion comprend de plus au moins une étiquette.

5. La méthode de la revendication 1, la revendication 2, la revendication 3 ou la revendication 4, dans laquelle la protéine de fusion comprend de plus au moins un site de clivage de protéine.

6. L'utilisation de YebF pour diriger la sécrétion d'une protéine, d'un polypeptide ou d'un peptide d'intérêt dans du milieu de croissance bactérienne, dans laquelle YebF est fusionné de façon amino-terminale à la protéine, au polypeptide ou au peptide d'intérêt.

7. L'utilisation de la séquence de tête de YebF pour diriger le transport d'une protéine, d'un peptide ou d'un polypeptide d'intérêt vers le périplasme, dans laquelle la séquence de tête de YebF est fusionnée de façon amino-terminale à la protéine, au polypeptide ou au peptide d'intérêt.

8. Une méthode pour exprimer une protéine, un polypeptide ou un peptide d'intérêt dans le périplasme, laquelle méthode comprend :
(a) fournir un vecteur d'expression qui code pour une protéine de fusion comprenant la protéine, le polypeptide ou le peptide d'intérêt situé(e) de façon carboxy-terminale par rapport à la séquence de tête de YebF, et
(b) exprimer la protéine dans une cellule bactérienne adéquate.

9. La méthode de la revendication 8, dans laquelle le vecteur d'expression est lié de façon opérationnelle pour contrôler des séquences nucléotidiques qui dirigent la transcription et la synthèse de la séquence de tête de YebF.

10. La méthode de la revendication 9, dans laquelle la séquence nucléotidique qui code pour la protéine, le polypeptide ou le peptide d'intérêt est située en aval de la séquence nucléotidique qui code pour la séquence de tête de YebF.

11. La méthode de la revendication 10, dans laquelle la protéine de fusion comprend de plus au moins une étiquette.

12. La méthode de la revendication 10, dans laquelle la protéine de fusion comprend de plus au moins un site de clivage de protéine.

13. La méthode de la revendication 8, dans laquelle la cellule bactérienne est *Escherichia coli.*


### SEQUENCE LISTING

<110> Research Corporation Technologies, Inc.
<120> PROTEIN PRODUCTION METHOD UTILIZING YEBF
<130> P107928EPPC/DAN
<140> EP 05 774 845.1
   <141> 2005-08-12
<150> US 60/522,125
   <151> 2004-08-18
<160> 21
<170> PatentIn version 3.2
<210> 1
   <211> 118
   <212> PRT
   <213> Escherichia coli
<400> 1
   Met Lys Lys Arg Gly Ala Phe Leu Gly Leu Leu Leu Val Ser Ala Cys
   1               5                   10                  15
   Ala Ser Val Phe Ala Ala Asn Asn Glu Thr Ser Lys Ser Val Thr Phe
               20                  25                  30
   Pro Lys Cys Glu Asp Leu Asp Ala Ala Gly Ile Ala Ala Ser Val Lys
           35                  40                  45
   Arg Asp Tyr Gln Gln Asn Arg Val Ala Arg Trp Ala Asp Asp Gln Lys
       50                  55                  60
   Ile Val Gly Gln Ala Asp Pro Val Ala Trp Val Ser Leu Gln Asp Ile
   65                  70                  75                  80
   Gln Gly Lys Asp Asp Lys Trp Ser Val Pro Leu Thr Val Arg Gly Lys
                   85                  90                  95
   Ser Ala Asp Ile His Tyr Gln Val Ser Val Asp Cys Lys Ala Gly Met
               100                 105                 110
   Ala Glu Tyr Gln Arg Arg
           115
<210> 2
   <211> 97
   <212> PRT
   <213> Escherichia coli
<400> 2
   Ala Asn Asn Glu Thr Ser Lys Ser Val Thr Phe Pro Lys Cys Glu Asp
   1               5                   10                  15
   Leu Asp Ala Ala Gly Ile Ala Ala Ser Val Lys Arg Asp Tyr Gln Gln
                   20                  25                  30
   Asn Arg Val Ala Arg Trp Ala Asp Asp Gln Lys Ile Val Gly Gln Ala
           35                  40                  45
   Asp Pro Val Ala Trp Val Ser Leu Gln Asp Ile Gln Gly Lys Asp Asp
       50                  55                  60
   Lys Trp Ser Val Pro Leu Thr Val Arg Gly Lys Ser Ala Asp Ile His
   65                  70                  75                  80
   Tyr Gln Val Ser Val Asp Cys Lys Ala Gly Met Ala Glu Tyr Gln Arg
                   85                  90                  95
   Arg
<210> 3
   <211> 357
   <212> DNA
   <213> Escherichia coli
<400> 3
   atgaaaaaaa gaggggcgtt tttagggctg ttgttggttt ctgcctgcgc atcagttttc     60
   gctgccaata atgaaaccag caagtcggtc actttcccaa agtgtgaaga tctggatgct    120
   gccggaattg ccgcgagcgt aaaacgtgat tatcaacaaa atcgcgtggc gcgttgggca    180
   gatgatcaaa aaattgtcgg tcaggccgat cccgtggctt gggtcagttt gcaggacatt    240
   cagggtaaag atgataaatg gtcagtaccg ctaaccgtgc gtggtaaaag tgccgatatt    300
   cattaccagg tcagcgtgga ctgcaaagcg ggaatggcgg aatatcagcg gcgttaa       357
<210> 4
   <211> 29
   <212> DNA
   <213> Artificial
<220>
   <223> Forward primer for amplification of the yeb F gene
<400> 4
   gagaattcgg agaaaaacat gaaaaaaag 29
<210> 5
   <211> 25
   <212> DNA
   <213> Artificial
<220>
   <223> Reverse primer for amplification of yeb F gene
<400> 5
   atatctcgag acgccgctga tattc 25
<210> 6
   <211> 43
   <212> DNA
   <213> Artificial
<220>
   <223> Primer for amplification of the alpha-amylase gene of B. subtilis X-23
<400> 6
   tagaattcag gagaaaaaca tggtcgactc ggtcaaaaac ggg 43
<210> 7
   <211> 25
   <212> DNA
   <213> Artificial
<220>
   <223> Primer for amplification of the alpha-amylase gene of B. subtilis X-23
<400> 7
   atatctcgag atgaggcgca tttcc 25
<210> 8
   <211> 25
   <212> DNA
   <213> Artificial
<220>
   <223> Primer for amplification of the leader sequence for fusing the sec-leader sequence of the yeb F gene to the mature alpha-amylase gene
<400> 8
   atatctcgag agcgaaaact gatgc 25
<210> 9
   <211> 27
   <212> DNA
   <213> Artificial
<220>
   <223> Primer for amplification of the hIL-2 gene
<400> 9
   gatcatgtcg acgctccgac ctccagc 27
<210> 10
   <211> 25
   <212> DNA
   <213> Artificial
<220>
   <223> Primer for amplification of the hIL-2 gene
<400> 10
   atatctcgag ggtcagggtg gagat 25
<210> 11
   <211> 133
   <212> PRT
   <213> Artificial
<220>
   <223> Amino acid sequence for the mature hIL-2 gene
<400> 11
<210> 12
   <211> 29
   <212> DNA
   <213> Artificial
<220>
   <223> Primer for the PCR-amplification of the phoA gene
<400> 12
   gcatatctcg agcggacacc agaaatgcc 29
<210> 13
   <211> 26
   <212> DNA
   <213> Artificial
<220>
   <223> Primer for the PCR-amplification of the phoA gene
<400> 13
   cgatagtcga ctttcagccc cagagc 26
<210> 14
   <211> 21
   <212> PRT
   <213> Escherichia coli
<400> 14
   Met Lys Lys Arg Gly Ala Phe Leu Gly Leu Leu Leu Val Ser Ala Cys
   1 5 10 15
   Ala Ser Val Phe Ala
   20
<210> 15
   <211> 21
   <212> PRT
   <213> shigella flexneri
<400> 15
   Met Lys Lys Arg Gly Ala Phe Leu Gly Arg Leu Leu Val Ser Ala Cys
   1 5 10 15
   Ala Ser Val Phe Ala
   20
<210> 16
   <211> 21
   <212> PRT
   <213> salmonella typhi
<400> 16
   Met Asn Lys Arg Gly Ala Leu Leu Ser Leu Leu Leu Leu Ser Ala Ser
   1 5 10 15
   Val Ser Ala Phe Ala
   20
<210> 17
   <211> 23
   <212> PRT
   <213> Yersinia pestis
<400> 17
   Met Lys Lys Thr Gly Leu Ala Leu Val Leu Ala Thr Ile Leu Leu Gly
   1 5 10 15
   Met Met Gly Ser Val His Ala
   20
<210> 18
   <211> 27
   <212> PRT
   <213> Pseudomonas luminescens
<400> 18
   Met Lys Thr Cys His Ile Ile Asn Arg Val Gly Leu Ser Gly Val Ala
   1 5 10 15
   Leu Leu Leu Thr Val Ser Phe Thr Val Ser Ala
   20 25
<210> 19
   <211> 4347
   <212> DNA
   <213> Artificial
<220>
   <223> The sequence of pYebFH6/MS
<400> 19
   tgttgacaat taatcatcgg ctcgtataat gtgtggaatt gtgagcggat aacaatttca 60
   cacaggaaac agaattagga gatagaattc atgaaaaaaa gaggggcgtt tttagggctg 120
   ttgttggttt ctgcctgcgc atcagttttc gctgccaata atgaaaccag caagtcggtc 180
   actttcccaa agtgtgaaga tctggatgct gccggaattg ccgcgagcgt aaaacgtgat 240
   tatcaacaaa atcgcgtggc gcgttgggca gatgatcaaa aaattgtcgg tcaggccgat 300
   cccgtggctt gggtcagttt gcaggacatt cagggtaaag atgataaatg gtcagtaccg 360
   ctaaccgtgc gtggtaaaag tgccgatatt cattaccagg tcagcgtgga ctgcaaagcg 420
   ggaatggcgg aatatcagcg gcgtctcgag caccaccacc accaccactg agatctccga 480
   tcctctagag tcgatcgacc tgcagccaag cttctgtttt ggcggatgag agaagatttt 540
   cagcctgata cagattaaat cagaacgcag aagcggtctg ataaaacaga atttgcctgg 600
   cggcagtagc gcggtggtcc cacctgaccc catgccgaac tcagaagtga aacgccgtag 660
   cgccgatggt agtgtggggt ctccccatgc gagagtaggg aactgccagg catcaaataa 720
   aacgaaaggc tcagtcgaaa gactgggcct ttcgttttat ctgttgtttg tcggtgaacg 780
   ctctcctgag taggacaaat ccgccgggag cggatttgaa cgttgcgaag caacggcccg 840
   gagggtggcg ggcaggacgc ccgccataaa ctgccaggca tcaaattaag cagaaggcca 900
   tcctgacgga tggccttttt gcgtttctac aaactctttt gtttattttt ctaaatacat 960
   tcaaatatgt atccgctcat gagacaataa ccctgataaa tgcttcaata atattgaaaa 1020
   aggaagagta tgagtattca acatttccgt gtcgccctta ttcccttttt tgcggcattt 1080
   tgccttcctg tttttgctca cccagaaacg ctggtgaaag taaaagatgc tgaagatcag 1140
   ttgggtgcac gagtgggtta catcgaactg gatctcaaca gcggtaagat ccttgagagt 1200
   tttcgccccg aagaacgttt tccaatgatg agcactttta aagttctgct atgtggcgcg 1260
   gtattatccc gtgttgacgc cgggcaagag caactcggtc gccgcataca ctattctcag 1320
   aatgacttgg ttgagtactc accagtcaca gaaaagcatc ttacggatgg catgacagta 1380
   agagaattat gcagtgctgc cataaccatg agtgataaca ctgcggccaa cttacttctg 1440
   acaacgatcg gaggaccgaa ggagctaacc gcttttttgc acaacatggg ggatcatgta 1500
   actcgccttg atcgttggga accggagctg aatgaagcca taccaaacga cgagcgtgac 1560
   accacgatgc ctgtagcaat ggcaacaacg ttgcgcaaac tattaactgg cgaactactt 1620
   actctagctt cccggcaaca attaatagac tggatggagg cggataaagt tgcaggacca 1680
   cttctgcgct cggcccttcc ggctggctgg tttattgctg ataaatctgg agccggtgag 1740
   cgtgggtctc gcggtatcat tgcagcactg gggccagatg gtaagccctc ccgtatcgta 1800
   gttatctaca cgacggggag tcaggcaact atggatgaac gaaatagaca gatcgctgag 1860
   ataggtgcct cactgattaa gcattggtaa ctgtcagacc aagtttactc atatatactt 1920
   tagattgatt taaaacttca tttttaattt aaaaggatct aggtgaagat cctttttgat 1980
   aatctcatga ccaaaatccc ttaacgtgag ttttcgttcc actgagcgtc agaccccgta 2040
   gaaaagatca aaggatcttc ttgagatcct ttttttctgc gcgtaatctg ctgcttgcaa 2100
   acaaaaaaac caccgctacc agcggtggtt tgtttgccgg atcaagagct accaactctt 2160
   tttccgaagg taactggctt cagcagagcg cagataccaa atactgtcct tctagtgtag 2220
   ccgtagttag gccaccactt caagaactct gtagcaccgc ctacatacct cgctctgcta 2280
   atcctgttac cagtggctgc tgccagtggc gataagtcgt gtcttaccgg gttggactca 2340
   agacgatagt taccggataa ggcgcagcgg tcgggctgaa cggggggttc gtgcacacag 2400
   cccagcttgg agcgaacgac ctacaccgaa ctgagatacc tacagcgtga gctatgagaa 2460
   agcgccacgc ttcccgaagg gagaaaggcg gacaggtatc cggtaagcgg cagggtcgga 2520
   acaggagagc gcacgaggga gcttccaggg ggaaacgcct ggtatcttta tagtcctgtc 2580
   gggtttcgcc acctctgact tgagcgtcga tttttgtgat gctcgtcagg ggggcggagc 2640
   ctatggaaaa acgccagcaa cgcggccttt ttacggttcc tggccttttg ctggcctttt 2700
   gctcacatgt tctttcctgc gttatcccct gattctgtgg ataaccgtat taccgccttt 2760
   gagtgagctg ataccgctcg ccgcagccga acgaccgagc gcagcgagtc agtgagcgag 2820
   gaagcggaag agcgcctgat gcggtatttt ctccttacgc atctgtgcgg tatttcacac 2880
   cgcacgaacg ccagcaagac gtagcccagc gcgtcggcca gcttgcaatt cgcgctaact 2940
   tacattaatt gcgttgcgct cactgcccgc tttccagtcg ggaaacctgt cgtgccagct 3000
   gcattaatga atcggccaac gcgcggggag aggcggtttg cgtattgggc gccagggtgg 3060
   tttttctttt caccagtgag acgggcaaca gctgattgcc cttcaccgcc tggccctgag 3120
   agagttgcag caagcggtcc acgtggtttg ccccagcagg cgaaaatcct gtttgatggt 3180
   ggttaacggc gggatataac atgagctgtc ttcggtatcg tcgtatccca ctaccgagat 3240
   atccgcacca acgcgcagcc cggactcggt aatggcgcgc attgcgccca gcgccatctg 3300
   atcgttggca accagcatcg cagtgggaac gatgccctca ttcagcattt gcatggtttg 3360
   ttgaaaaccg gacatggcac tccagtcgcc ttcccgttcc gctatcggct gaatttgatt 3420
   gcgagtgaga tatttatgcc agccagccag acgcagacgc gccgagacag aacttaatgg 3480
   gcccgctaac agcgcgattt gctggtgacc caatgcgacc agatgctcca cgcccagtcg 3540
   cgtaccgtct tcatgggaga aaataatact gttgatgggt gtctggtcag agacatcaag 3600
   aaataacgcc ggaacattag tgcaggcagc ttccacagca atggcatcct ggtcatccag 3660
   cggatagtta atgatcagcc cactgacgcg ttgcgcgaga agattgtgca ccgccgcttt 3720
   acaggcttcg acgccgcttc gttctaccat cgacaccacc acgctggcac ccagttgatc 3780
   ggcgcgagat ttaatcgccg cgacaatttg cgacggcgcg tgcagggcca gactggaggt 3840
   ggcaacgcca atcagcaacg actgtttgcc cgccagttgt tgtgccacgc ggttgggaat 3900
   gtaattcagc tccgccatcg ccgcttccac tttttcccgc gttttcgcag aaacgtggct   3960
   ggcctggttc accacgcggg aaacggtctg ataagagaca ccggcatact ctgcgacatc   4020
   gtataacgtt actggtttca cattcaccac cctgaattga ctctcttccg ggcgctatca   4080
   tgccataccg cgaaaggttt tgcaccattc gatggtgtca acgtaaatgc cgcttcgcct   4140
   tcgcgcgcga attgcaagct gatccggagc ttatcgactg cacggtgcac caatgcttct   4200
   ggcgtcaggc agccatcgga agctgtggta tggctgtgca ggtcgtaaat cactgcataa   4260
   ttcgtgtcgc tcaaggcgca ctcccgttct ggataatgtt ttttgcgccg acatcataac   4320
   ggttctggca aatattctga aatgagc                                       4347
<210> 20
   <211> 20
   <212> PRT
   <213> Escherichia coli
<400> 20
   Met Lys Lys Arg Gly Ala Phe Leu Gly Leu Leu Leu Val Ser Ala Cys
   1               5                   10                  15
   Ala Ser Val Phe
               20
<210> 21
   <211> 21
   <212> PRT
   <213> salmonella typhimurium
<400> 21
   Met Asn Lys Arg Gly Ala Leu Leu Ser Leu Leu Phe Leu Ser Ala Ser
   1               5                   10                  15
   Val Ser Ala Phe Ala
               20